# NEUE EUROPÄISCHE PATENTSCHRIFT

(11) **EP 0 415 296 B2**
(45) Veröffentlichungstag und Bekanntmachung des Hinweises auf die Entscheidung über den Einspruch: **25.04.2001**
(45) Hinweis auf die Patenterteilung: 02.10.1996
(21) Anmeldenummer: 90116322.0
(22) Anmeldetag: 25.08.1990
(51) Int. Cl.: C12N 9/54, C12N 15/57, C11D 3/386, C12N 1/21

(54) **Neue hochalkalische Proteasen**
High alkaline proteases
Protéases particulièrement alcalines

(30) Priorität: 31.08.1989 DE 3928804; 24.07.1990 DE 4023458
(43) Veröffentlichungstag der Anmeldung: 06.03.1991
(73) Patentinhaber: Genencor International GmbH, 31582 Nienburg/Weser (DE); Gesellschaft für Biotechnologische Forschung mbH (GBF), D-38124 Braunschweig (DE)
(72) Erfinder: Vetter, Roman, D-3167 Burgdorf (DE); Wilke, Detlef, D-3015 Wennigsen (DE); Amory, Antoine, B-1330 Rixensart (BE); Clippe, André, B-1200 Bruxelles (BE); Aehle, Wolfgang, D-3300 Branschweig (DE); Schomburg, Dietmar, D-3300 Branschweig (DE)
(74) Vertreter: Brandes, Jürgen, Dr. rer. nat.

(56) Entgegenhaltungen:
- WO-A-89/06279
- WO-A-91/00345
- US-A- 3 770 587
- TRENDS IN BIOCHEMICAL SCIENCES, vol. 13, August 1988, Seiten 291-297; J.A. WELLS et al: "Subtilisin - an enzyme designed to be engineered"
- DNA, Band 6, Nr. 6, Dezember 1987, Seiten 583-591; J. BOTTERMAN et al.
- T.E. Creighton"Proteins, Structure and Molecular Principles" W.H. Freeman & Co., N.Y. 1984
- Bode et al. EMBO J. 1986, Vol. 5, p.813-818

## Beschreibung

Die vorliegende Erfindung bezieht sich auf, durch gerichtete Mutagenese von für hochalkalische Proteasen codierenden DNA-Sequenzen, optimierte hochalkalische Proteasen, auf DNA-Sequenzen (Gene), die für diese Proteasen codieren, auf Vektoren, die diese DNA-Sequenzen enthalten und auf mit diesen Vektoren transformierte Mikroorganismen, auf ein Verfahren zur Herstellung von optimierten hochalkalischen Proteasen, sowie auf die optimierte Proteasen enthaltende Waschmittel.

Hochalkalische Proteasen sind wertvolle industrielle Produkte mit vorteilhaften Anwendungen, insbesondere in der Waschmittelindustrie, da sie Protein enthaltende Verunreingungen entfernen. Um wirksam zu sein, müssen diese Proteasen nicht nur proteolytische Aktivität unter Waschbedingungen (pH-Wert, Temperatur) besitzen, sondern müssen darüber hinaus auch mit anderen Waschmittelbestandteilen, d.h. in Kombination mit anderen Enzymen, Tensiden, Gerüststoffen (Builder), Bleichmitteln, Bleichmittelaktivatoren und anderen Zusatz- und Hilfsstoffen verträglich sein, d. h. diesen gegenüber ausreichende Stabilität und in Gegenwart dieser Substanzen ausreichende Wirksamkeit aufweisen.

Hochalkalische Proteasen sind spezielle Enzyme, die gewonnen werden durch Kultivierung von Mikroorganismen, insbesondere durch Kultivierung von Bacillus Spezies, die, wie z.B. Bacillus alcalophilus, die gewünschte hochalkalische Protease produzieren und in das Kulturmedium ausscheiden, aus welchem die Protease isoliert werden kann. Diese hochalkalischen Proteasen unterscheiden sich von gewöhnlichen alkalischen Proteasen, wie sie durch Kultivierung von Bacillus-Spezies, wie insbesondere z.B. B. subtilis, B. amyloliquefaciens und B. licheniformis, gewonnen werden können.

Im Stand der Technik wurden zwar bereits viele Anstrengungen unternommen, um neue hochalkalische Proteasen mit gewünschten Eigenschaften zu erzielen, und es sind bereits eine Reihe von natürlichen und künstlich (gentechnisch) veränderten alkalischen und hochalkalischen Proteasen bekannt.

Beispielsweise wird in der EP 328 229 A1 ein Enzymprodukt vorgeschlagen, welches mutierte proteolytische Enzyme umfaßt, die durch Expression eines Gens dieses Enzymes erhalten werden, wobei sich die Aminosäurensequenz dieses mutierten Enzyms wenigstens in einer Aminosäure von dem Wild-Typ-Enzym unterscheidet und sich durch verbesserte Eigenschaften für die Anwendung in Detergenzien auszeichnet. Insbesondere werden Proteasen mit wenigstens 70 % Homologie zur Aminosäurensequenz der PB92-Serinprotease vorgeschlagen, die sich durch eine Mutation in einer der Positionen 116, 126, 127, 128, 160, 166, 169, 212 oder 216 von der Wild-Typ-Protease unterscheidet.

Je doch besteht immer noch Bedarf an neuen, optimierten hochalkalischen Proteasen, insbesondere im Hinblick auf die Wascheigenschaften optimierten hochalkalischen Proteasen.

Es bestand daher die Aufgabe, neue wertvolle hochalkalische Proteasen mit optimierten Eigenschaften herzustellen, die dafür benötigten DNA-Sequenzen (Gene) durch gerichtete Mutagenese zu erzeugen und die benötigten Vektoren und transformierten Mikroorganismen zur Verfügung zu stellen.

Die Aufgabe wird gelöst durch die erfindungsgemäßen hochalkalischen Proteasen, durch die zugehörigen DNA-Sequenzen (Gene), durch das erfindungsgemäße Verfahren, sowie durch die Waschmittel, enthaltend die erfindungsgemäßen Proteasen.

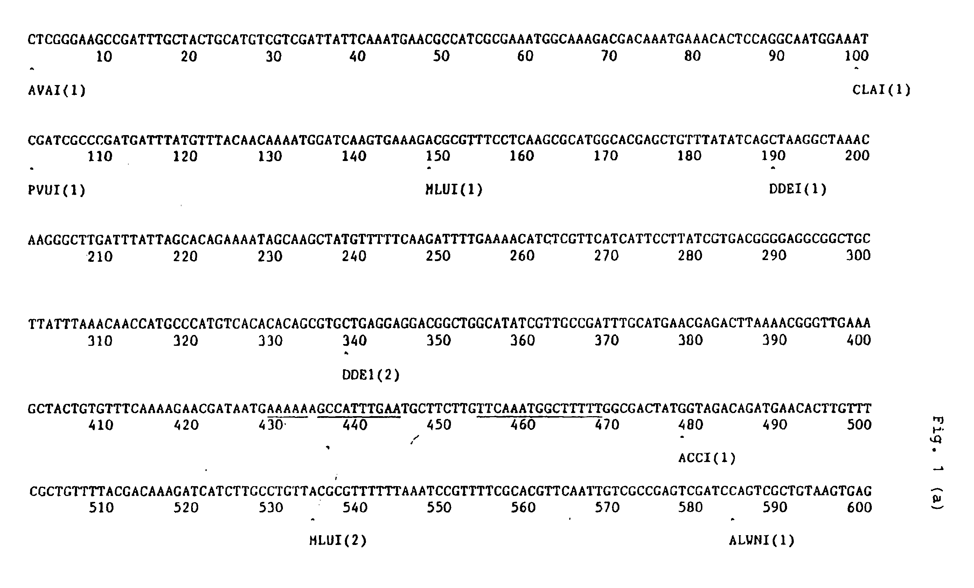

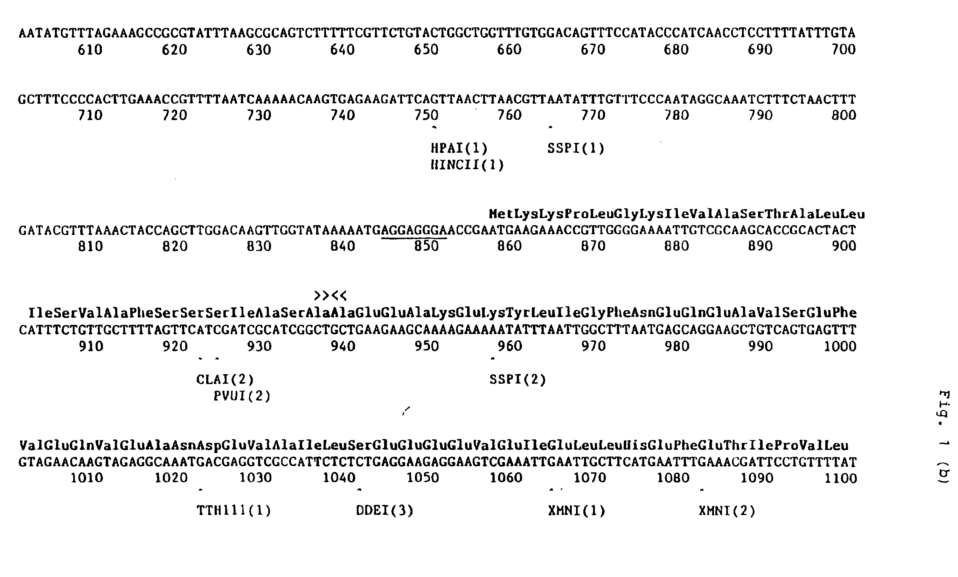

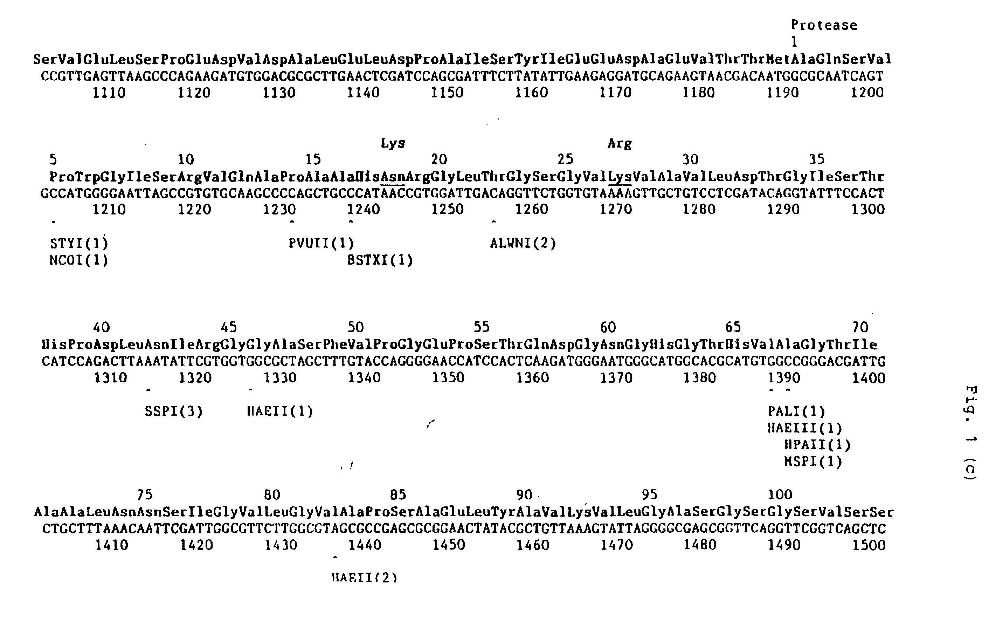

Die Erfindung betrifft (bezüglich des Vertragsstaates Griechenland) hochalkalische Proteasen, welche sich dadurch auszeichnen, daß sie eine Aminosäurensequenz aufweisen, welche wenigstens 80 % Homologie zu der in Fig. 1 angegebenen Aminosäurensequenz besitzt und sich von dieser in mindestens einer der Positionen 18, 27, 42, 57, 114, 115, 135, 138, 188, 189, 238, 255, 266 der Fig. 1, vorzugsweise 18, 27, 42, 57, 114, 115, 135, 138, 238, 255, 266, oder in einer der dazu homologen Positionen dadurch unterscheidet, daß die in der betreffenden Position befindliche Aminosäure durch die stärker basische Aminosäure Lysin oder Arginin ausgetauscht ist, wobei diese hochalkalischen Proteasen Molekulargewichte von 26000 bis 28000 g/mol, gemessen durch SDS-Polyacrylamid-Gelelektrophorese gegenüber Referenzproteinen mit bekanntem Molekulargewicht, besitzen sich weiterhin durch ein pH-Optimum im Bereich von 10 bis 12,5 auszeichnen, wobei unter pH-Optimum derjenige pH-Bereich verstanden wird, in dem die Proteasen maximale proteolytische Aktivität aufweisen.

Mit Blick auf die verbliebenen und benannten Vertragsstaaten und die Offenbarung der WO-A-91/00345 (Artikel 54(3) (4) EPÜ) werden erfindungsgemäß hochalkalische Proteasen, die diese codierende DNA-Sequenzen, Waschmittel, die die Protease enthalten und ein Verfahren zur Herstellung einer optimierten hochalkalischen Protease bzw. (bezüglich des Vertragsstaates Spanien) Verfahren zur Herstellung der Protease, Verfahren zur Herstellung der DNA-Sequenzen und Waschmittel beansprucht, wobei die Protease dadurch gekennzeichnet sind, daß sie ein pH-Optimum von 10 bis 12,5 und ein Molekulargewicht von 26000 bis 28000 g/mol besitzt, und daß sie eine Aminosäuresequenz aufweist, welche wenigstens 80% Homologie zu der in Fig. 1 angegebenen Aminosäuresequenz besitzt und sich von dieser
- in mindestens einer der Positionen 18, 57, 114, 115, 135, 188, 189, 238, 255 der Fig. 1, vorzugsweise 18, 57, 114, 115, 135, 238, 255, oder in einer der dazu homologen Positionen dadurch unterscheidet, daß die in der betreffenden Position befindliche Aminosäure durch die stärker basische Aminosäure Lysin oder Arginin ausgetauscht ist, oder
- in Position 266 oder in einer dazu homologen Position dadurch unterscheidet, daß die in der betreffenden Position befindliche Aminosäure durch die Aminosäure Lysin ausgetauscht ist.

Die erfindungsgemäßen Proteasen weisen eine gegenüber der Ausgangsprotease im wesentlichen unveränderte, d.h. gleich gute pH-Stabilität auf. In einer bevorzugten Ausgestaltung der Erfindung liegen solche Proteasen vor, deren Aminosäurensequenzen eine Homologie von über 90 %, insbesondere aber von über 95 %, zu der Aminosäurensequenz der Fig. 1 besitzen und bei denen mindestens eine Aminosäure in einer der angegebenen Positionen durch eine stärker basische Aminosäure ausgetauscht ist.

Unter Homologie zu der in Fig. 1 angebenen Aminosäurensequenz wird hier eine strukturell sehr enge Verwandtschaft der betreffenden Aminosäurensequenzen zu der in Fig. 1 angebenen Aminosäurensequenz verstanden. Zur Bestimmung der Homologie werden jeweils die einander strukturell entsprechenden Abschnitte der Aminosäurensequenz der Fig. 1 und der damit zu vergleichenden Aminosäurensequenz so zur Deckung miteinander gebracht, daß maximale Strukturübereinstimmung zwischen den Aminosäurensequenzen besteht, wobei durch Deletion oder Insertion einzelner Aminosäuren verursachte Unterschiede berücksichtigt und durch entsprechende Verschiebungen von Sequenzabschnitten ausgeglichen werden. Die Zahl der nunmehr in den Sequenzen miteinander übereinstimmenden Aminosäuren ("homologe Positionen") bezogen auf die Gesamtzahl der in der Sequenz der Fig. 1 enthaltenen Aminosäuren gibt dabei die Homologie in % an. Abweichungen in den Sequenzen können sowohl durch Variation, Insertion als auch Deletion von Aminosäuren bedingt sein.

Es versteht sich dementsprechend von selbst, daß bei den erfindungsgemäßen hochalkalischen Proteasen, die aus zur Figur 1 wenigstens zu 80 % homologen Proteasen gewonnen wurden, sich die mit Bezug auf die Fig. 1 bezeichneten Aminosäurenpositionen auf die dazu homologen Positionen der erfindungsgemäßen Proteasen beziehen. Deletionen oder Insertionen in den zu Fig. 1 homologen Proteasen können zu einer relativen Verschiebung der Aminosäurenpositionen führen, so daß in homologen Teilstücken von zueinander homologen Aminosäurensequenzen die numerischen Bezeichnungen der einander entsprechenden Aminosäurepositonen nicht identisch zu sein brauchen.

Die Zahl der in eine Protease eingeführten Mutationen ist keinen Beschränkungen unterworfen. In den erfindungsgemäß optimierten Proteasen können insbesondere Aminosäuren in einer oder mehreren, beispielsweise in 1 bis 3, vorzugsweise in 1 oder 2 der vorgenannten Positionen gegen stärker basische Aminosäuren ausgetauscht sein. Beispiele von erfindungsgemäßen hochalkalischen Proteasen, in denen in mehr als nur einer Position Aminosäuren in den vorgenannten Positionen gegen stärker basische Aminosäuren ausgetauscht wind, sind z.B. solche Proteasen mit wenigstens 80 % Homologie zu der in Figur 1 angegebenen Aminosäurensequenz, in welcher in zwei der genannten Positionen, z.B. in Position 27 und 115 bzw. 27 und 135 die betreffende Aminosäure gegen eine der stärker basichen Aminosäuren Lysin oder Arginin ausgetausch ist.

Als gegen die in den Austauschpositionen ursprünglich befindlichen Aminosäure auszutauschende stärker basische Aminosäuren erweist sich insbesondere Arginin als Zweckmäßig. Als besonders zweckmäßige Beispiele von erfindungsgemäß optimierten hochalkalischen Proteasen seien einerseits Proteasen genannt, in denen die Aminosäure in Position 18 gegen Lysin ausgetauscht ist, und andererseits Proteasen, bei denen die Aminosäure in Position 27, 42, 57, 114, 115, 135, 138, 238, 255 oder 266 gegen Arginin ausgetauscht ist.

Zur Gewinnung der erfindungsgemäßen Proteasen werden Mikroorganismen kultiviert, die mit einem Expressionsvektor transformiert wurden, der das für die betreffende Protease codierende Strukturgen enthält; die Expressionsvektoren wurden hierbei durch weiter unten beschriebene Verfahren gewonnen. Die Bereitstellung dieser Verfahren umfaßt auch die Sequenzierung der Protease der Fig. 1.

Die Erfindung umfaßt daher auch solche transformierten Mikroorganismen, Expressionsvektoren und andere Vektoren, sowie Proteasestrukturgene (d.h. für die Protease codierende DNA-Sequenzen), die für Verfahren zur Sequenzierung der Protease der Fig. 1 oder für die Konstruktion und Gewinnung der erfindungsgemäßen hochalkalischen Proteasen von besonderer Bedeutung sind.

Die erfindungsgemäßen DNA-Sequenzen zeichnen sich dadurch aus, daß sie für eine hochalkalische Protease codieren, die eine Aminosäurensequenz aufweist, welche wenigstens 80 % Homologie zu der in Fig. 1 angegebenen Aminosäurensequenz besitzt und sich von dieser in mindestens einer der Positionen 18, 27, 42, 57, 114, 115, 135, 138, 188, 189, 238, 255, 266 der Fig. 1, vorzugsweise 18, 27, 42, 57, 114, 115, 135, 138, 238, 255, 266, oder in einer der dazu homologen Positionen dadurch unterscheidet, daß die in der betreffenden Position befindliche Aminosäure durch die stärker basische Aminosäure Lysin oder Arginin ausgetauscht ist. Bevorzugt sind solche erfindungsgemäßen DNA-Sequenzen, die für hochalkalische Proteasen codieren, deren Aminosäurensequenzen eine Homologie von über 90 %, insbesondere aber von über 95 %, zu der Aminosäurensequenz der Fig. 1 besitzen und bei denen mindestens eine Aminosäure in einer der angegebenen Positionen durch eine stärker basische Aminosäure ausgetauscht ist. Die erfindungsgemäßen DNA-Sequenzen können insbesondere für vorstehende Aminosäurensequenzen codieren, in denen Aminosäuren in 1 bis 3, vorzugsweise in 1 oder 2, der vorgenannten Positionen gegen stärker basische Aminosäuren ausgetauscht sind. Beispiele für durch diese erfindungsgemäßen DNA-Sequenzen codierte Proteasen, in denen mehr als eine, z.B. zwei, Aminosäuren ausgetauscht sind, sind weiter oben für die entsprechenden erfindungsgemäßen hochalkalischen Proteasen bereits angegeben.

Die erfindungsgemäßen DNA-Sequenzen zeichnen sich also dadurch aus, daß sie für hochalkalische Proteasen codieren, bei welchen die betreffende Aminosäure gegen die stärker basische Aminosäure Lysin oder Arginin, insbesondere jedoch gegen Arginin, ausgetauscht ist. Als besonders zweckmäßige Beispiele von erfindungsgemäßen DNA-Sequenzen seien einerseits DNA-Sequenzen genannt, welche für hochalkalische Proteasen codieren, in denen die Aminosäure in Position 18 gegen Lysin ausgetauscht ist, und andererseits DNA-Sequenzen, die für hochalkalische Proteasen codieren, in denen die Aminosäure in Position 27, 42, 57, 114, 115, 135, 138, 238, 255 oder 266 gegen Arginin ausgetauscht ist.

Die vorstehend beschriebenen erfindungsgemäßen DNA-Sequenzen sind beispielsweise in den Vektoren enthalten, die für die Transformation von Mikroorganismen geeignet sind. Diese Vektoren können insbesondere Expressionsvektoren sein, die sich für die Transformation von solchen Mikroorganismen eignen, die für die Herstellung und Gewinnung hochalkalischer Proteasen eingesetzt werden können; für eine großtechnische Produktion der erfindungsgemäßen Proteasen können die vorstehend beschriebenen, erfindungsgemäßen DNA-Sequenzen zweckmäßigerweise auch in das Genom des zur Produktion dienenden Mikroorganismus integriert werden.

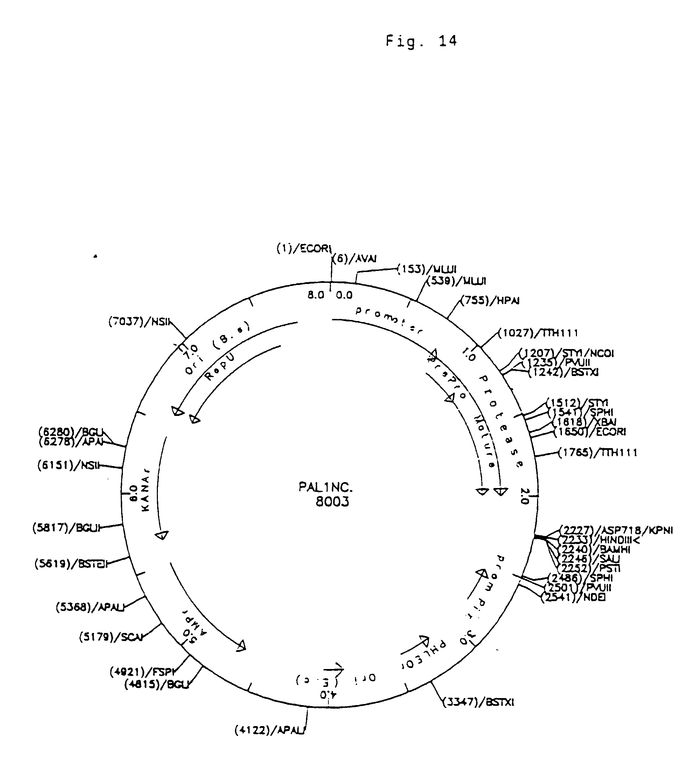

Bevorzugte Vektoren sind Plasmide. Eine Gruppe hier verwendeter Plasmide enthält DNA-Sequenzen aus E. coli, die für beta-Lactamase (Marker; z.B. Ampicillinresistenz) und für den E. coli "origin of replication" (enthält die für eine Vermehrungsfähigkeit des Plasmides in E. coli benötigten Erbinformationen) codieren, DNA-Sequenzen, die für Antibiotikaresistenz (Marker; z.B. Kanamycin- oder Neomycinresistenz) und für den Bacillus "origin of replication" (enthält die für eine Vermehrungsfähigkeit des Plasmides in Bacillus-Spezies benötigten Erbinformationen) in Bacillus subtilis codieren, die Promotorsequenz und die Preprosequenz für die erfindungsgemäße Protease, und eine der erfindungsgemäßen DNA-Sequenzen. Solche Plasmide, die nach den weiter unten beschriebenen Verfahren erhältlich sind, eignen sich hervorragend als Expressionsvektoren. Ein Beispiel für einen solchen Expressionsvektor mit der Bezeichnung pAL1NC gibt die Restriktionskarte der Fig. 14 wieder.

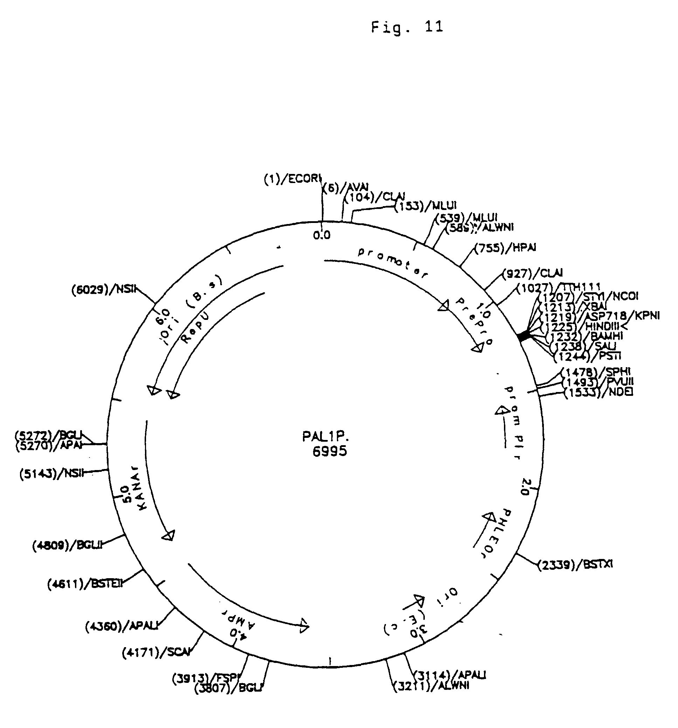

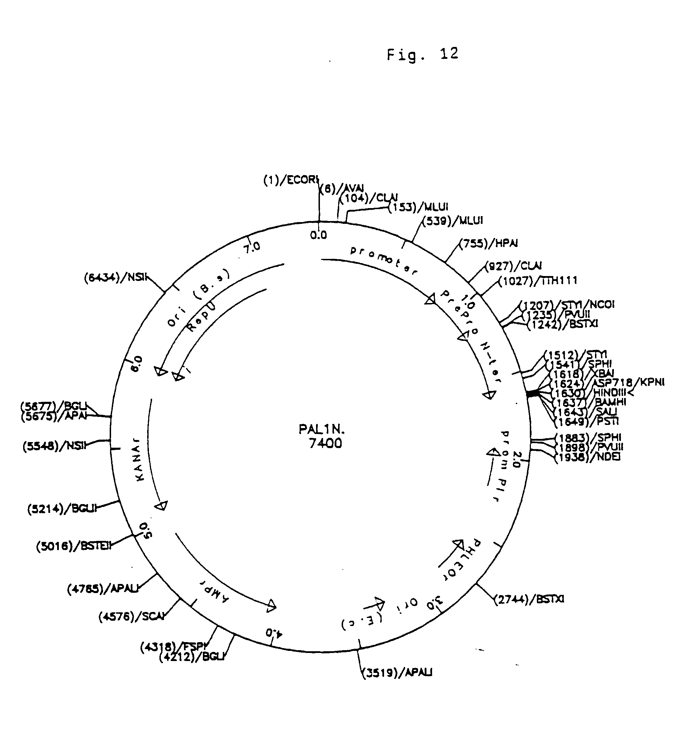

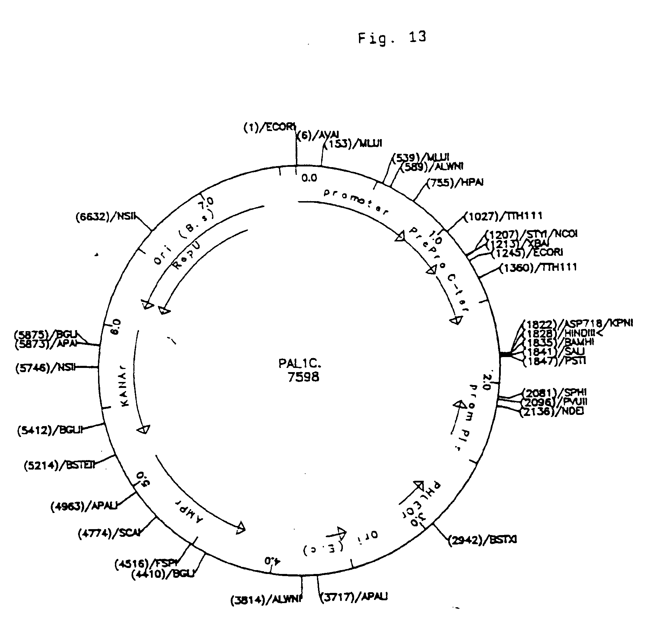

Zu den im Rahmen der Erfindung verwendeten Vektoren zählen weiterhin Vektoren, welche Vorläufer der vorstehend beschriebenen, Expressionsvektoren darstellen. In einer Variante enthalten diese alle in dem vorstehend beschriebenen Expressionsvektor enthaltenen DNA-Sequenzen mit Ausnahme der erfindungsgemäßen mutierten DNA-Sequenz, welche für die erfindungsgemäße Protease codiert. Die Stelle der Protease-DNA-Sequenz ist hier mit einem synthetischen Linker besetzt, der sich durch Schneiden mit geeigneten Restriktionsendonukleasen ganz oder teilweise entfernen und in einer anschließenden Rekombination des verbliebenen Vektorteiles mit der Protease-DNA-Sequenz durch diese ersetzen läßt. Ein Beispiel für einen solchen Expressionsvektor-Vorläufer mit der Bezeichnung pAL1 P gibt die Restriktionskarte der Fig. 11 wieder. Der synthetische Linker reicht in diesem Beispiel von der Restriktionsstelle Ncol (1207), über die Restriktionsstellen Xbal (1213) und Asp718 (1219) bis zur Restriktionsstelle Hindlll (1225). In einer anderen Variante enthalten die Expressionsvektor-Vorläufer zusätzlich bereits auch solche Teile der ProteaseDNA-Sequenzen, in denen keine Mutationen vorgenommen werden sollen. Ein solcher Vektor enthält z.B. die N- oder C-terminale Hälfte der Protease-DNA, je nachdem ob die Mutation der Protease-DNA durch Austausch einer Aminosäure in der C-terminalen oder der N-terminalen Hälfte des Proteasestrukturgens erfolgt. Solche Expressionsvektor-Vorläufer werden aus dem vorstehenden Expressionsvektor-Vorläufer des Typs pAL1 P erhalten, indem man einen Teil des synthetischen Linkers durch die N- oder die C-terminale Hälfte des nicht-mutierten Proteasestrukturgens austauscht. Beispiele für diese Vorläufer des vollständigen Expressionsvektors mit der Bezeichnung pAL1 N (enthält die nicht-mutierte N-terminale Hälfte des Proteasestrukturgens, die das Ncol/Xbal-Fragment des synthetischen Linkers im Vektor pAL1 P ersetzt) bzw. mit der Bezeichnung pAL1 C (enthält die nicht-mutierte C-terminale Hälfte des Protease-strukturgens, die das Xbal/Asp718-Fragment des synthetischen Linkers im Vektor pAL1 P ersetzt) geben die Restriktionskarten der Fig. 12 bzw. der Fig. 13 wieder.

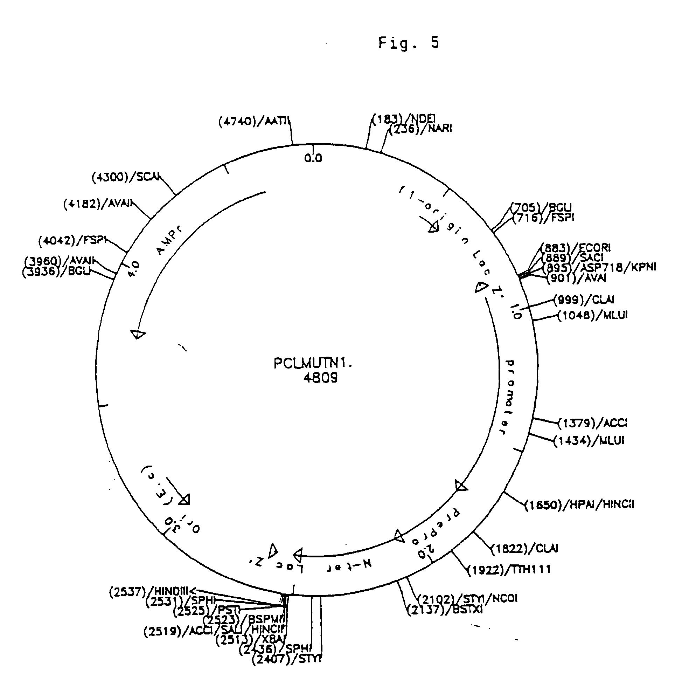

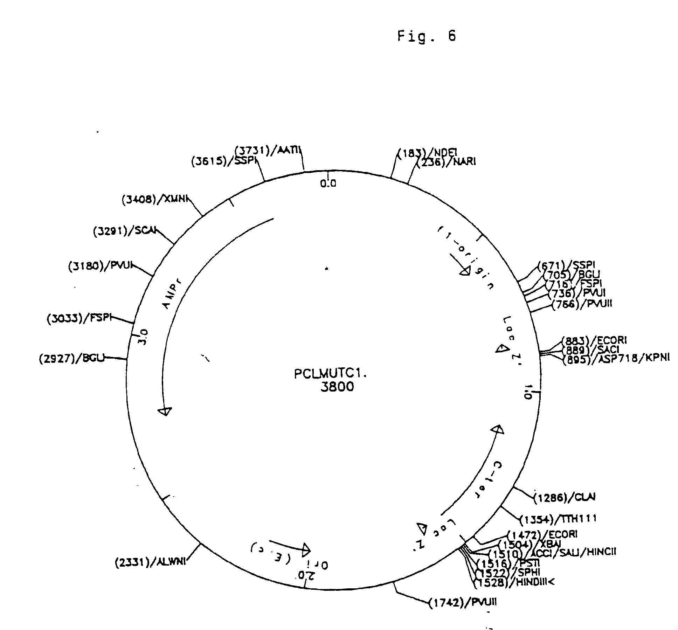

Zu den im Rahmen der Erfindung verwendeten Vektoren zählt weiterhin eine Gruppe von Phagemiden, welche vorteilhaft zur Gewinnung von mutierten N- bzw. C-terminalen Hälften des Proteasestrukturgenes eingesetzt wird. Es handelt sich z.B. um Phagemide, in die durch Schneiden mit geeigneten Restriktionsendonukleasen und nachfolgende Rekombination mit den entsprechenden Hälften des Proteasestrukturgens der hochalkalischen Ausgangsprotease entweder die N-terminale Hälfte inklusive des zum Proteasegen gehörigen Promotors und der Prepro-DNA-Sequenzen oder die C-terminale Hälfte des Proteasestrukturgens eingebaut wurden. Beispiele für diese Vektoren mit der Bezeichnung pCLMUTN1 (enthält die nicht-mutierte N-terminale Hälfte des Strukturgens der hochalkalischen Ausgangsprotease einschließlich der zugehörigen Prepro- und Promotorsequenzen) bzw. mit der Bezeichnung pCLMUTC1 (enthält die nicht-mutierte C-terminale Hälfte des Strukturgens der hochalkalischen Ausgangsprotease) geben die Fig. 5 und die Fig. 6 wieder.

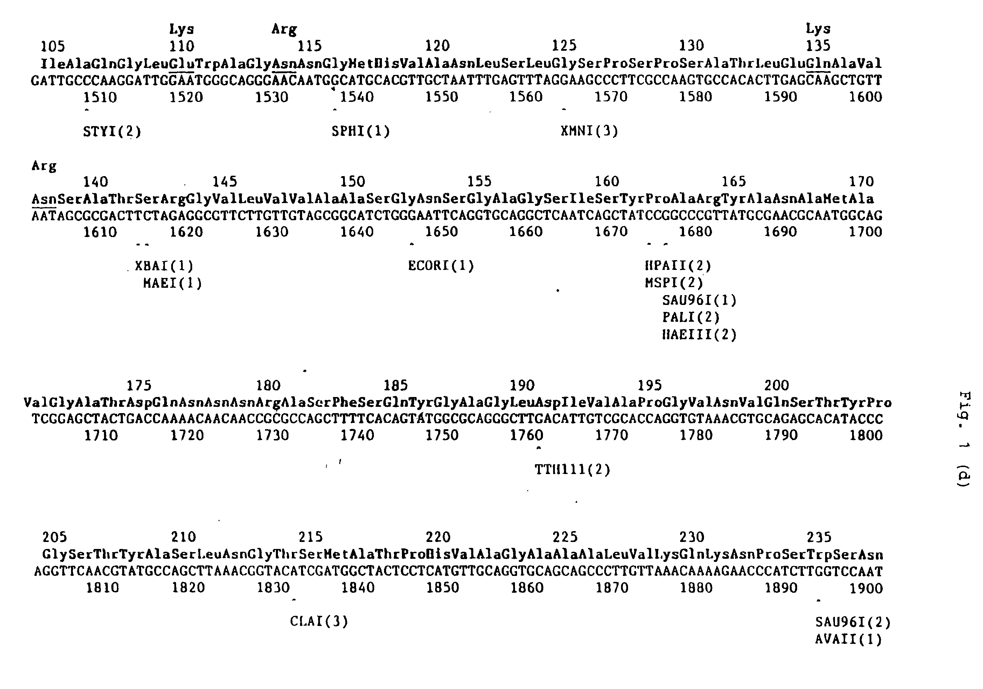

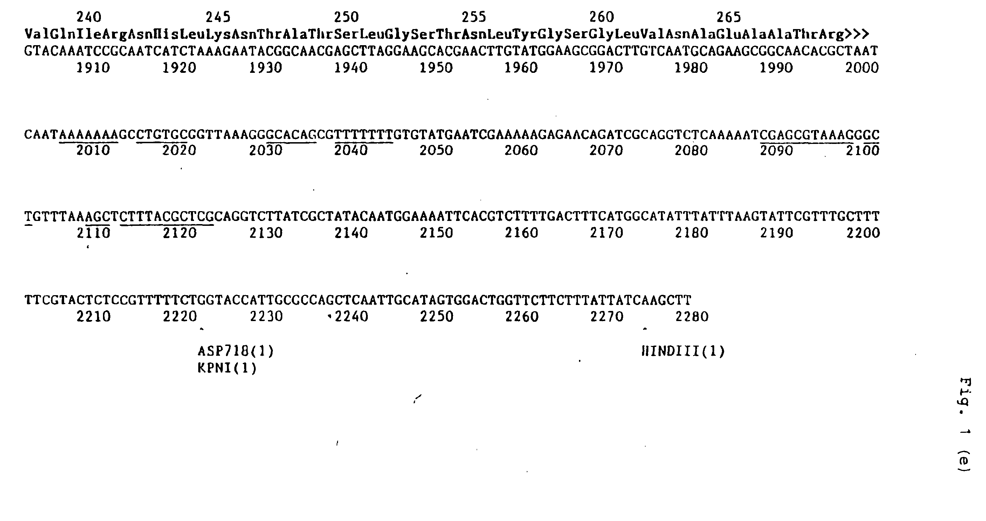

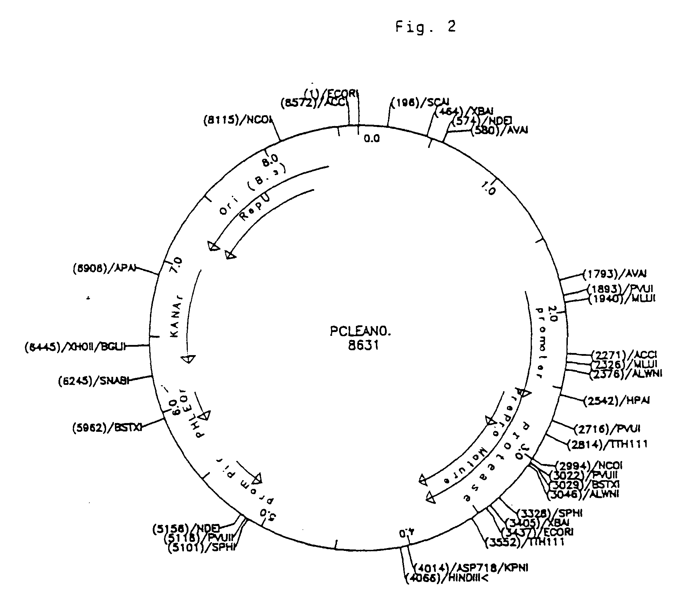

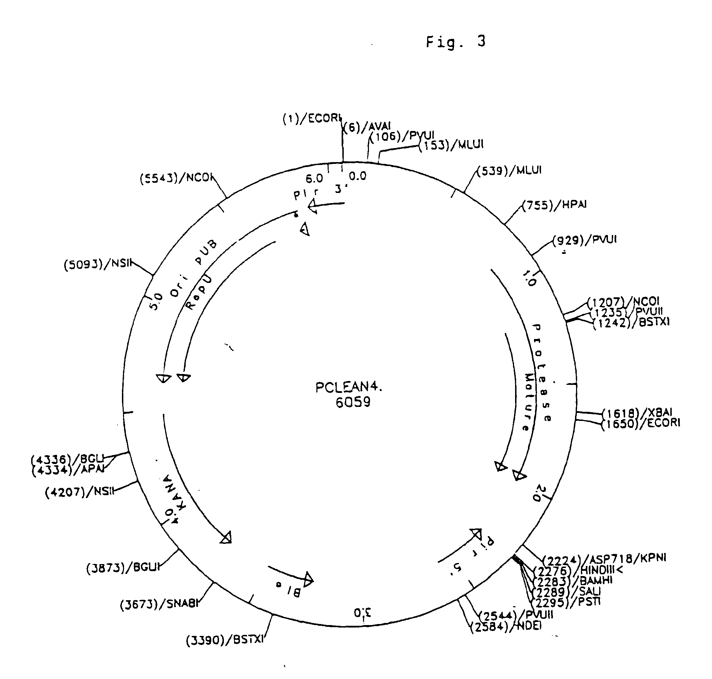

Zu den im Rahmen der Erfindung verwendeten Vektoren zählt weiterhin eine Gruppe von Plasmiden, welche Klonierungs- und Expressionsvektoren umfaßt, die einerseits für die Isolierung, Replikation und Sequenzierung des Strukturgens der hochalkalischen Ausgangsprotease von Bedeutung sind und andererseits als Quelle für das gesamte Proteasestrukturgen oder für Teile davon, die für die Konstruktion der oben beschriebenen Vektoren benötigt werden, dienen. Diese Vektoren enthalten DNA-Sequenzen, die für Antibiotikaresistenz und für den "origin of replication° in Bacillus codieren, sowie Promotor-, Prepro- und DNA-Sequenzen der hochalkalischen Ausgangsprotease. Beispiele für solche Plasmide mit der Bezeichnung pCLEAN0 bzw. pCLEAN4 sind in den Restriktionskarten der Fig. 2 und Fig. 3 wiedergegeben.

Die im Rahmen der Erfindung verwendeten Mikroorganismen zeichnen sich dadurch aus, daß sie mit einem der oben beschrieben Vektoren transformiert sind. Geeignet sind an sich alle Mikroorganismen, die die Information der beschriebenen Vektoren exprimieren können. Zu den hier verwendeten Mikroorganismen gehören einerseits zur Produktion der erfindungsgemäßen Protease geeignete transormierte Mikroorganismen und anderseits auch solche transformierten Mikroorganismen, welche im Rahmen der nachstehend beschriebenen Verfahren der Sequenzierung und der gerichteten DNA-Sequenz-Mutagenese erstmals erzeugt wurden. Zur Gewinnung der transformierten Mikroorganismen für die Protease-Produktion können Mikroorganismen benutzt werden, die sich zur Transformation mit einem Expressionsvektor und zur Produktion von hochalkalischen Proteasen eignen. Hierzu gehören bereits an sich Protease produzierende Bakterien, vorzugsweise Bacillus-Spezies, aber auch Mikroorganismen wie z.B. Bakterien, die sich zur Transformation mit dem beschriebenen Expressionsvektor eignen, von sich aus aber noch keine Protease produzieren, sondern diese Fähigkeit erst durch die Transformation erlangen. Besonders bevorzugt sind an sich bereits alkalische oder hochalkalische Protease produzierende Bakterien bzw. deren Protease-defiziente Mutanten, die mit einem der hier verwendeten Expressionsvektoren transformiert sind; solche Bakterien sind insbesondere Bacillus-Spezies, wie Bacillus subtilis, Bacillus alcalophilus, Bacillus licheniformis und Bacillus amyloliquefaciens. Andere Mikroorganismen sind z.B. mit einem der hier verwendeten Vektoren transformierte Bakterien der Spezies E. coli. Diese Mikroorganismen eignen sich insbesondere für den Einsatz in Verfahren zur Konstruktion der im Rahmen der vorliegenden Erfindung verwendeten Vektoren, wobei diese Mikroorganismen zur Selektion und Replikation der Vektoren dienen, sowie für den Einsatz in Verfahren zur Sequenzierung und der gerichteten Mutagenese, in denen sie zur Gewinnung einzelsträngiger DNA-Sequenzen dienen.

Weiterhin umfaßt die Erfindung Waschmittel, die wenigstens eine der erfindungsgemäßen hochalkalischen Proteasen enthalten. Für diesen Anwendungszweck stellt die Erfindung eine Gruppe neuer hochalkalischer Proteasen mit einzelnen gegenüber vorbekannten Proteasen verbesserten Eigenschaften zur Verfügung, aus welcher je nach den speziel benötigen Eigenschaften (Waschleistung, Temperaturbeständigkeit, Verträglichkeit mit anderen Bestandteilen) der einzusetzenden hochalkalischen Protease, eine für die jeweilige spezielle Waschmittelformulierung besonders geeignete erfindungsgemäße Protease ausgewählt werden kann. Die erfindungsgemäßen Proteasen können in Waschmittelformulierungen, insbesondere in Pulverwaschmittelformulierungen, einzeln oder gewünschtenfalls auch in Kombination miteinander, gegebenenfalls auch in Kombination mit Waschmittelproteasen des Standes der Technik oder anderen an sich üblichen Waschmittelenzymen, wie z.B. Amylasen, Lipasen, Pektinasen, Nukleasen, Oxidoreduktasen etc., eingesetzt werden. Die erfindungsgemäßen Proteasen werden in den Waschmittelformulierungen in an sich für Waschmittelenzyme üblichen Mengen, insbesondere in Mengen von bis zu 3 Gew.-% (bezogen auf die Trokkensubstanz der Gesamtzusammensetzung), vorzugsweise in einer Menge von 0,2 bis 1,5 Gew-%, verwendet.

Außer den bereits erwähnten Waschmittelenzymen können die Waschmittel der Erfindung alle an sich im Stand der Technik üblichen Waschmittelinhaltsstoffe wie Tenside, Bleichmittel oder Gerüststoffe (Builder), sowie weitere übliche Hilfsstoffe für die Formulierung von Waschmitteln in an sich üblichen Mengen enthalten. Zu den Hilfsstoffen gehören z.B. Verstärker, Enzymstabilisatoren, Schmutzträger und/oder Kompatibilisierungsmittel, Komplex- und Chelatbildner, Seifenschaumregulatoren und Zusatzstoffe wie optische Aufheller, Opazifizierungsmittel, Korrosionsinhibitoren, Antielektrostatika, Farbstoffe, Bakterizide, Bleichmittelaktivatoren, Persäurebleichmittelvorstufen.

So enthalten erfindungsgemäße Waschmittelformulierungen in typischer beispielhafter Zusammensetzung bezogen auf Trockensubstanz
a) wenigstens 5 Gew.-%, z.B. 10 bis 50 Gew.-%, eines Tensids oder Tensidgemisches
b) zu 40 Gew.-% eines Builders oder eines Builder-Gemisches,
c) bis zu 40 Gew.-% eines Bleichmittels oder Bleichmittelgemisches, vorzugsweise ein Perborat wie Natriumperborat-Tetrahydrat oder Natriumperborat-Monohydrat,
d) bis zu 3 Gew.-% wenigstens einer erfindungsgemäßen Protease und
e) weitere Bestandteile wie Hilfsstoffe etc. ad 100 Gew.-%

Solche Waschmittelformulierungen können in an sich üblicher Weise formuliert werden. Die erfindungsgemäßen Proteasen können dazu z.B. in Form von Granulaten, Prills oder Pellets, gegebenenfalls auch mit Oberflächenüberzügen versehen, mit den anderen Komponenten der Waschmittelformulierung in an sich bekannter Weise vermischt werden.

Die Erfindung umfaßt ferner ein Verfahren zur Herstellung einer erfindungsgemäß optimierten hochalkalischen Protease mit einem erfindungsgemäß transformierten Mikroorganismus, der einen Vektor mit einer DNA-Sequenz enthält, die für eine Aminosäurensequenz codiert, welche wenigstens 80 % Homologie zu der in Fig. 1 angegebenen Aminosäurensequenz der Ausgangsprotease besitzt und sich von dieser in mindestens einer der Positionen 18, 27, 42, 57, 114, 115, 135, 138, 188, 189, 238, 255, 266 der Fig. 1, vorzugsweise 18, 27, 42, 57, 114, 115, 135, 138, 238, 255, 266 oder in einer der dazu homologen Positionen dadurch unterscheidet, daß die in der betreffenden Position befindliche Aminosäure gegen die stärker basische Aminosäure Lysin oder Arginin ausgetauscht ist. Der erfindungsgemäß transformierte Mikroorganismus wird kultiviert und aus dem Kulturmedium eine optimierte hochalkalische Protease mit einer gegenüber der Aminosäurensequenz der Ausgangsprotease der Fig. 1 in mindestens einer der vorgenannten Positionen durch Austausch einer Aminosäure gegen eine der stärker basischen Aminosäuren Lysin oder Arginin veranderten Aminosäurensequenz isoliert. Vorzugsweise werden in diesem Verfahren solche Mikroorganismen eingesetzt, die einen Vektor mit einer DNA-Sequenz enthalten, die für eine Aminosäurensequenz einer hochalkalischen Protease codiert, wobei die Aminosäurensequenz dieser Protease eine Homologie von über 90 %, insbesondere von über 95 %, zu der Aminosäurensequenz der Fig, 1 besitzt und in der Aminosäurensequenz die Aminosäure in wenigstens einer der angegebenen Positionen durch die stärker basische Aminosäure Lysin oder Arginin ausgetauscht ist.

Zur Erzeugung der im vorstehenden Verfahren eingesetzten Mikroorganismen kann so vorgegangen werden, daß man
a) zunächst aus einem geeigneten Bakterium, welches eine hochalkalische Protease mit einer Aminosäurensequenz mit mindestens 80 %, vorzugsweise über 90 %, insbesondere aber über 95 % Homologie zu der Aminosäurensequenz der Fig. 1 produziert, die für die Protease codierende DNA-Sequenz (d.h. das Strukturgen der Protease) isoliert,
b) die Nukleotidabfolge dieser DNA-Sequenz bestimmt,
c) in der nunmehr bekannten DNA-Sequenz solche Mutationen (Punktmutationen) erzeugt, daß die mutierte DNA-Sequenz nun für eine hochalkalische Protease, in der Aminosäuren der Ursprungsprotease in vorstehend gegebenen Positionen gegen eine stärker basische Aminosäure ausgetauscht sind, codiert,
d) nachfolgend mit Hilfe der mutierten DNA-Sequenz einen Expressionsvektor erzeugt und
e) den erhaltenen Expressionsvektor in einen geeigneten Mikroorganismus, welcher schließlich zur Herstellung der mutierten hochalkalischen Protease eingesetzt werden kann, transformiert.

Die Verfahrensschritte zur Konstruktion und Gewinnung der erfindungsgemäßen hochalkalischen Proteasen, sowie die hierbei erhaltenen Zwischenprodukte in Form DNA-Sequenzen, Vektoren, insbesondere Expressionsvektoren, und transformierten Mikroorganismen werden nachfolgend im einzelnen näher beschrieben.

Die Strukturgene, die für Aminosäurensequenzen hochalkalischer Proteasen mit wenigstens 80 % Homologie zu der in Fig. 1 angegebenen Aminosäurensequenz codieren, können nach an sich bekannten, allgemeinen Methoden erhalten werden. Hierzu wird z.B. aus einem Bakterium ("Donor-Bakterium"), insbesondere aus einer Bacillus-Spezies, die die hochalkalische Protease produziert, die chromosomale DNA nach an sich bekannten Methoden isoliert und mit geeigneten Restriktionsendonukleasen partiell hydrolysiert. Restriktionsendonukleasen sind Enzyme, die substratsspezifisch doppelsträngige DNA dadurch in Fragmente zerlegen, daß sie Phosphatdiesterbindungen zwischen einzelnen Nukleotidbausteinen der DNA spalten. Alle Restriktionsendonukleasen vermögen bestimmte Basensequenzen der DNA zu erkennen, welche für die Aktivität der betreffenden Restriktionsendonukleasen spezifische Wirkungsorte (Schnittstellen) markieren. Beim Schneiden (Restriktion) doppelsträngiger DNA entstehen bei einigen Restriktionsendonukleasen spezifische, sogenannte "überstehende Enden", die unter bestimmten Renaturierungsbedingungen wieder miteinander oder mit entsprechenden (komplementären) überstehenden Enden anderweitig gewonnener DNA-Fragmente verbunden (ligiert) werden können (Rekombination). Beim Schneiden mit anderen Restriktionsendonukleasen entstehen DNA-Doppelstränge mit glatten Enden. Diese DNA-Doppelstränge mit glatten Enden können mit beliebigen DNA-Doppelsträngen, die ebenfalls glatte Enden besitzen, rekombiniert werden.

Die erhaltenen Restriktionsfragmente der Donor-DNA können z.B. durch Gelelektrophorese nach Größe aufgetrennt und die Fragmente gewünschter Größe dann mit einer geeigneten, doppelsträngigen Vektor-DNA rekombiniert werden.

Vektoren sind DNA-Moleküle, die sich als Transportmoleküle (Vehikel) zur Einschleusung (Transformation) von Fremd-DNA in Wirtszellen eignen, dort ggf. autonom replizierbar sind und gegebenenfalls noch sogenannte Marker besitzen. Marker sind DNA-Fragmente, die für bestimmte beobachtbare Eigenschaften (z.B. Antibiotika-Resistenz) codieren und der nachfolgenden Selektion der transformierten Mikroorganismen (Transformanten) dienen. Häufig verwendete Vektoren sind die sogenannten Plasmide, d.h. extrachromosomale, ringförmige, doppelsträngige BakterienDNA, die sich durch geeignete Methoden in andere Mikroorganismen einbringen läßt und dort vermehrbar ist.

Mit der in vitro rekombinierten DNA (Vektor + Restriktionsfragmente der Donor-DNA) können Bakterien, vorzugsweise eine Bacillus-Spezies, transformiert werden und die Transformanten nach der bekannten Markereigenschaft (z. B. Neomycin-Resistenz) selektiert werden. Man erhält so Klone, d.h. genetisch identische Transformanten. Unter diesen Transformanten können solche, die vermehrt Protease ausscheiden, auf proteinhaitigen Platten gesucht und danach isoliert werden. Aus einem Klon mit Proteaseaktivität wird schließlich die in diesen Transformanten eingeführte Plasmid-DNA isoliert und durch erneute Transformation eines Bakteriums überprüft, ob die Proteaseaktivität Plasmidgebunden ist, d.h. ob die Proteaseaktivität mit der Markereigenschaft gekoppelt ist.

Das so isolierte Plasmid enthält neben der Vektor-DNA mit bekannten Restriktionsstellen das gewünschte Strukturgen für die zu optimierende hochalkalische Ausgansprotease und weitere, hier aber nicht benötigte DNA-Sequenzen aus dem Donor-Bakterium. Ein Beispiel für einen solchen Vektor mit der Bezeichnung pCLEAN0 gibt die Restriktionskarte der Fig. 2 wieder.

Um den Aufwand für die nachfolgende Sequenzierung des Strukturgens der zu optimierenden hochalkalischen Protease möglichst gering zu halten, empfiehlt es sich, vor der eigentlichen Sequenzierung die zusätzlichen, nicht benötigten DNA-Sequenzen aus der Donor-DNA-Sequenz zu eleminieren und die Donor-DNA-Sequenz im wesentlichen auf das Strukturgen für die Protease zu reduzieren. Hierzu wird z.B. das Plasmid, welches das Strukturgen und die zusätzliche DNA-Sequenz umfaßt, mit einer Anzahl verschiedener Restriktionsendonukleasen geschnitten (restringiert), die erhaltenen DNA-Fragmente durch Gelelektrophorese nach Größe getrennt und anhand des gefundenen Bandenmusters eine Restriktionskarte erstellt. Es werden so die Restriktionsstellen, die im Bereich der Donor-DNA-Sequenz angesiedelt sind, ermittelt. Die Kenntnis der Restriktionskarte des Plasmids ermöglicht es nunmehr, aus diesem durch Schneiden mit ausgewählten Restriktionsendonukleasen ein DNA-Fragment aus der Donor-DNA-Sequenz herauszuschneiden, welches im wesentlichen nur noch das Strukturgen für die hochalkalische Protease, die zugehörigen Pre- und Pro-Einheiten, sowie die für die Genexpression benötigte Promotor-Einheit umfaßt.

Durch den Wiedereinbau dieser in der Größe reduzierten Donor-DNA-Sequenz in einen geeigneten Vektor kann ein neuer, replizierbarer Vektor erhalten werden, dessen Fähigkeit zur Expression der hochalkalischen Ausgangsprotease überprüft werden kann, indem man ein Bakterium, insbesondere eine Bacillus-Spezies, mit diesem Vektor transformiert, den erhaltenen Transformanten kultiviert und auf Proteaseaktivität überprüft. Ein Beispiel für einen solchen reduzierten Vektor mit der Bezeichnung pCLEAN4 gibt die Restriktionskarte der Fig. 3 wieder.

Zur Bestimmung der Nukleotidsequenz (Sequenzierung) des Proteasestrukturgens wird zunächst der vorstehend beschriebene Vektor in einem geeigneten Mikroorganismus repliziert, und das Proteasegen isoliert. Dieses wird sodann in einen Phagemiden subkloniert und die erhaltenen Phagemiden anschließend in einen geeigneten Mikroorganismus z.B. E. coli transformiert und durch Kultivierung der Transformanten einzelsträngige, das Proteasegen enthaltende DNA produziert. Die gebildete einzelsträngige DNA wird isoliert und der Sequenzierung zugeführt. Die Sequenzierung wird nach an sich bekannten Methoden ausgeführt, indem man z.B. die Einzelstrang-DNA mit dem Proteasegen nach Maxam und Gilbert einer basenspezifischen partiellen chemischen Spaltung zuführt (1980, in Methods in Enzymology, Grossmann L., Moldave K., eds., Academic Press Inc., New York und London, Vol. 65, 499), oder indem man z.B. die Einzelstrang-DNA mit dem Proteasegen als Matritze für die partielle Synthese von Teilstücken des komplementären DNA-Stranges nach der Dideoxy-Kettenterminator-Methode nach Sanger und Brownlee (1977, Proc. Natl. Acad. Sci. USA 74: 5473) einsetzt.

Die ermittelte Nukleotidsequenz kann nunmehr mit Hilfe des genetischen Codes (ein Triplett-Wort = Codon steht für eine definierte Aminosäure) in die Aminosäurensequenz der Protease übersetzt werden. Zur Bestimmung des Anfangspunktes der Aminosäurensequenz des reifen Protease-Enzyms (d.h. das Enzym ohne die Pre- und Pro-Einheiten) wird am N-terminalen Ende der reifen Protease ein kurzes Stück der Aminosäurenabfolge durch an sich bekannte Methoden zur Bestimmung von Aminosäurensequenzen in Peptiden bestimmt. Die bekannte N-terminale Aminosäurensequenz kann nun anhand des genetischen Codes dem entsprechenden Teilstück der obigen Nukleotidsequenz zugeordnet werden und so der Anfangspunkt der für die reife Protease codierenden DNA-Sequenz festgelegt werden. Die weitere Aminosäurenabfolge der Protease ergibt sich dann zwangsläufig aus der DNA-Sequenz durch Zuordnung der nachfolgenden Aminosäuren mit Hilfe des genetischen Codes.

Erfindungsgemäß wird die für die Protease codierende DNA-Sequenz durch Austausch der entsprechenden Codons derart mutiert, daß die mutierte DNA-Sequenz für eine optimierte hochalkalische Protease codiert, in welcher in mindestens einer der Positionen 18, 27, 42, 57, 114, 115, 135, 138, 188, 189, 238, 255, 266 der Aminosäurensequenz in Fig. 1, vorzugsweise 18, 27, 42, 57, 114, 115, 135, 138, 238, 255, 266, oder in einer der dazu homologen Positionen die betreffende Aminosäure gegen die stärker basische Aminosäure Lysin oder Arginin ausgetauscht ist.

Die erfindungsgemäß austauschbaren Aminosäuren befinden sich in solchen Positionen in Oberflächenbereichen des Proteasemoleküls, daß durch den Austausch das katalytische Zentrum der Protease und Zentren, welche für die Aufrechterhaltung der Sekundär- und Tertiärstruktur des Proteasemoleküls von Bedeutung sind, praktisch unbeeinflußt bleiben.

Erfindungsgemäß werden so optimierte hochalkalische Proteasen mit pH-Optima von 10 bis 12,5 bereitgestellt, welche z.B. eine gegenüber der Ausgangsprotease unveränderte pH-Stabilität, jedoch verbesserte Wascheigenschaften unter spezifischen Bedingungen (Waschmitteltype, Temperatur etc.) aufweisen.

Die Einführung der Punktmutationen in die für die hochalkalischen Proteasen codierende DNA wird durch an sich bekannte Methoden zur gerichteten Mutagenese bewerkstelligt. Hierzu wird aus geeigneten Vektoren (Phagemide), z.B. aus pCLMUTN1 der Fig. 5 oder pCLMUTC1 der Fig. 6, gegebenenfalls unter Mithilfe eines Helfer-Phagen, ringförmige Einzelstrang-DNA erzeugt, die das gesamte Strukturgen, oder aber vorzugsweise nur denjenigen Teil (z.B. nur den N-terminalen Teil bzw. den C-terminalen Teil) des Strukturgens der Ursprungsprotease, in welchem die Mutation vorgenommen werden soll, enthält. Mit dieser ringförmigen Einzelstrang-DNA hybridisiert man ein synthetisches, hybridisierfähiges Oligonukleotid, welches in der gewünschten Punktmutationsstelle einen Nukleotidbaustein enthält, welcher so ausgewählt ist, daß das zugehörige Codon für eine gegenüber der originären Aminosäure in dieser Position stärker basische Aminosäure Arginin oder Lysin codiert. Zusätzlich ist das Oligonukleotid gegenüber der zu hybridisierenden, originären Nukleotidsequenz noch derart durch einen oder einige wenige weitere Nukleotidbausteine abgewandelt, daß die Codierung der originären Aminosäurensequenz zwar im Rahmen der Degeneration des genetischen Codes erhalten bleibt, jedoch in der originären Protease-Nukleotidsequenz eine gegebenenfalls vorhandene Restriktionsstelle im synthetischen Oligonukleotid entfernt bzw eine weitere Restriktionsstelle in das synthetische Oligonukleotid eingeführt wird. Die entfernte bzw. eingeführte Restriktionsstelle dient später zur Identifizierung der Mutanten-DNA-Sequenz gegenüber der Ausgangstyp-DNA-Sequenz mit Hilfe geeigneter Restriktionsendonukleasen. In einer Variante wird im Verfahren der gerichteten Mutagenese uracylierte Einzelstrang-DNA als Matrize erzeugt und für die Hybridisierung mit den synthetischen Oligonukleotiden verwendet. Nach Beendigung der Reaktionen des Verfahrens der gerichteten Mutagenese kann der Uracil-haltige DNA-Einzelstrang, der als Matrize zur Erzeugung mutierter DNA-Stränge (Vektoren) diente, durch Behandlung mit Uracil-N-Glucosylase beseitigt werden, ohne daß es einer phänotypischen Selektion von Mutanten bedarf. Die Glucosylase-Behandlung kann sowohl mit dem isolierten Enzym als auch mit Hilfe eines geeigneten Mikroorganismus mit Uracil-N-Glucosylase-Aktivität durchgeführt werden, der mit mutierter Vektor-DNA transformiert wurde.

Die Ergänzung der durch Hybridisierung erhaltenen partiell doppelsträngige DNA-Sequenz zum vollständigen Doppelstrang wird dann durch Zugabe der benötigten Nukleotide und unter Einwirkung von DNA-Polymerase und DNA-Ligase durchgeführt. Die erzeugte ringförmige, doppelsträngige DNA-Sequenz wird nachfolgend als Vektor in einen geeigneten Mikroorganismus transformiert und nach ausreichender Replikation die mutierten DNA-Sequenzen überdie unitären Restriktionsendonukleasen-Erkennungsstellen identifiziert und anschließend isoliert. Wird uracylierte Einzelstrang-DNA eingesetzt, so wird die Replikation z.B. in einem E. coli-Stamm vorgenommen, der vorzugsweise den mutierten, nicht-uracylierten DNA-Strang des im Mutationsverfahren erzeugten Doppelstrang-Vektors vermehrt. Hierdurch wird die Selektion der mutierten DNA-Vektoren zusätzlich erleichtert.

Die für die gerichtete Mutagenese benötigten synthetischen Oligonukleotide werden nach an sich bekannten Methoden hergestellt. Beispielsweise kann die Herstellung der Oligonukleotide nach Beaucage S.L. und Caruthers M.H. (1981, Tetrahedron Letters 22: 1859 - 1862) mit β-Cyanoethyl-phosphoramidit in einem Cyclone-Synthetiser (Biosearch) erfolgen. Die erhaltenen Oligonukleotide können z.B. durch Elution aus Polyacrylamid-Gelen und gegebenenfalls anschließende Entsalzung mit Hilfe von Sephadex-Säulen gereinigt und der weiteren Verwendung zugeführt werden. Die synthetischen Oligonukleotide können direkt als Primer für die DNA-Polymerase im vorstehend beschriebenen Mutagenese-Verfahren dienen. Die synthetischen Oligonukleotidsequenzen umfassen z.B. 20 bis 30 Nukleotidbausteine, die für etwa 7 bis 10 Aminosäuren codieren. Es ist natürlich auch möglich längere Nukleotidsequenzen für die obige Hybridisierung einzusetzen, doch führt dies zu keinen weiteren Vorteilen, solange eine ausreichende Hybridisierungsfähigkeit der kurzkettigen synthetischen Oligonukleotide sichergestellt ist. Längere Nukleotidsequenzen kommen aber insbesondere dann in Frage, wenn zwei oder mehrere Mutationen in benachbarte Positionen eingeführt werden sollen. Das Oligonukleotid kann dann entweder als solches aus Mononukleotiden synthetisiert oder durch Synthese aus geeigneten kürzerern Oligonukleotidsequenzen hergestellt werden. Dieses ist jedoch nicht zwingend, da zwei oder mehrere Mutationen auch durch aufeinanderfolgende Mutationen, beispielsweise im N-terminalen oder im C-terminalen Teil der Protease-DNA, mit zwei oder mehreren geeigneten Oligonukleotidsequenzen durchgeführt werden können. Doppelmutationen können darüber hinaus auch durch Kombination von mutierten C-Terminalen und mutierten N-terminalen DNA-Fragmenten, die für das C-terminale bzw. das N-terminale Ende einer erfindungsgemäßen hochalkalischen Protease codieren, erzeugt werden.

Die durch das oben beschriebene Verfahren der gerichteten Mutagenese erhaltenen ringförmigen, doppelsträngigen DNA-Sequenzen mit den eingeführten Mutationen stellen mutierte Vektoren dar, aus denen durch Behandlung mit geeigneten Restriktionsendonukleasen je nach Fall das gesamte mutierte Protease-Strukturgen oder das mutierte Teilstück des Protease-Strukturgens herausgeschnitten und in einen geeigneten Expressionsvektor eingebracht (subkloniert) werden kann. Mit diesem Expressionsvektor werden dann geeignete Mikroorganismen, z.B. Bacillus-Spezies, transformiert, die nachfolgend zur Expression und Gewinnung der mutierten hochalkalischen Proteasen unter geeigneten Bedingungen kultiviert werden.

In einer bevorzugten Ausgestaltung der Erfindung wird nicht das gesamte Strukturgen für die gerichtete Mutagenese eingesetzt, sondern nur ein Teilstück desselben, in dem die Mutation erzeugt werden soll. Hierzu wird aus dem Vektor der zur Replikation der Strukturgene dient, z.B. die N-terminale oder C-terminale Hälfte des Strukturgens mit geeigneten Restriktionsendonukleasen herausgeschnitten und in einen passenden Phagemiden subkloniert. Man erhält so Vektoren, die entweder die N-terminale oder die C-terminale Hälfte des Strukturgens der Protease enthalten und die in einem geeigneten Mikroorganismus, z.B. E. coli, zunächst ausreichend repliziert und dann der oben beschriebenen, gerichteten Mutagenese zugeführt werden. Die Mutagenese von Teilstücken des Strukturgenes hat den Vorteil, daß kürzere Einzelstrang-DNA-Sequenzen verwendet werden können und somit nach dem Hybridisierungsschritt mit synthetischen Oligonukleotiden im partiellen DNA-Doppelstrang wesentlich weniger Nukleotide als bei Verwendung der gesamten DNA-Sequenz zu ergänzen sind. Hierdurch wird der synthetische Aufwand und zusätzlich die Gefahr unerwünschter zufälliger Mutationen reduziert. Darüber hinaus lassen sich durch spätere Kombination von mutierten N- und C-terminalen Hälften der Protease-DNA-Sequenz leicht Doppelmutationen in der Protease-DNA-Sequenz erzeugen.

Die mutierten DNA-Sequenzen können aus dem zur Erzeugung der Mutationen dienenden Klonierungsvektoren durch geeignete Restriktionsendonukleasen herausgeschnitten und in entsprechende Restriktionsstellen besitzende Vektoren eingebaut werden, die Vorläufer der eigentlichen, für die Expression der hochalkalischen Protease benötigten Expressionsvektoren darstellen. Diese Vektoren sind so aufgebaut, daß sie außer den geeigneten Restriktionsstellen (z.B. aus einem synthetischen Linker) auch bereits die für die Proteaseexpression in einem Wirtsorganismus benötigten regulatorischen Sequenzen, Signalsequenzen, Promotorsequenzen und die für die Pre-und Proeinheiten der Protease codierenden DNA-Sequenzen enthalten.

Durch die Subklonierung einer mutierten DNA-Sequenz in einen solchen Vektor wird der eigentliche Expressionsvektor für eine optimierte hochalkalische Protease erhalten. Der Einbau der mutierten DNA-Sequenz in diesen Vorläufer des Expressionsvektors erfolgt so, daß ein Expressionsvektor mit geeignetem Leseraster entsteht. Hierbei können mutierte Teilstücke der für die Protease codierenden DNA-Sequenz, z.B. ein C-terminales oder ein N-terminales Teilstück, in bereits das jeweils restliche nicht mutierte oder ggf. auch mutierte (Erzeugung von Mehrfachmutationen) Teilstück enthaltende Vektoren eingebaut werden; oder es wird die gesamte für die Protease codierende mutierte DNA-Sequenz in Vektoren, welche noch keine Teilstücke dieser Protease-DNA-Sequenz enthalten, eingebaut. Beispiele für solche, bereits ein Teilstück der nicht-mutierten oder ggf. auch bereits mutierten DNA-Sequenz enthaltende Vorläufervektoren eines Expressionsvektors sind die Vektoren mit der Bezeichnung pAL1N und pAL1C, deren Restriktionskarte in den Fig. 12 bzw. Fig. 13 wiedergegeben sind. Ein Vektor, der noch kein Teilstück der Protease-DNA-Sequenz enthält ist der Vektor pAL1P mit der in Fig. 11 angegebenen Restriktionskarte.

Die Expressionsvektoren-Vorläufer für die bevorzugte Variante der Erfindung (Mutation in der N-terminalen Hälfte oder in der C-terminalen Hälfte) werden z.B. wie folgt erhalten. Zunächst führt man in ein Bacillus-Plasmid eine Polyklonierungsstelle ein. Das so erhaltene Plasmid wird restringiert und mit einem E. coli-Plasmidfragment, welches Marker und für die Replikation wichtige Sequenzteile enthält, rekombiniert. Anschließend werden gegebenenfalls solche Restriktionsstellen z.B. durch gerichtete Mutagenese entfernt, die spätere Verfahrensschritte stören würden. Aus dem so erhaltenen Plasmid wird ein neuer Vektor konstruiert, der die aus dem Bacillus-Plasmid und dem E. coli-Plasmid zur Replikation dienenden DNA-Sequenzen, DNA-Sequenzen für den Promotor, DNA-Sequenzen, die für die PreProsequenz der Protease codieren (erhalten aus z.B. dem Plasmid pCLEAN4 der Fig. 3), und einen synthetischen Linker enthält. Ein Beispiel für ein solches Plasmid mit der Bezeichnung pAL1 P gibt die Restriktionskarte der Fig. 11 wieder. Der synthetische Linker ist dabei derart ausgewählt, daß nach Schneiden mit geeigneten Restriktionsendonukleasen entweder mit dem gesamten Ursprungsstrukturgen bzw. dem gesamten mutierten Strukturgen bzw. mit mutierten oder nicht mutierten Teilstücken des Strukturgens kombiniert werden kann. Zur Herstellung eines Expressionsvektor-Vorläufers, der z.B. mit einer mutierten N-terminalen Hälfte des Strukturgens rekombiniert werden soll, wird in den vorstehend konstruierten Vektor, der die genannten Bacillus-, E. coli-, die Promotor- und die Pre- und Prosequenzen der Protease sowie den synthetischen Linker enthält, durch Schneiden des synthetischen Linkers z.B. zunächst die nicht-mutierte oder ggf. auch eine bereits mutierte (z.B. für Erzeugung von Proteasen mit Mutationen im C- und N-terminalen Teil der Protease-DNA-Sequenz) C-terminale Hälfte des Strukturgens der Protease eingeführt. Man erhält so die bereits genannten Vektoren vom Typ pAL1C der Fig. 13. Anschließend wird durch nochmaliges Schneiden des synthetischen Linkers die noch fehlende, mutierte N-terminale Hälfte des Protease-Strukturgens eingeführt. Auf diese Weise erhält man einen Vektor vom Typ pAL1 NC der Fig. 14. Analoges gilt für den umgekehrten Fall. Es wird dann zunächst die nicht-mutierte oder ggf. auch eine bereits mutierte N-terminale Hälfte in einen Vektor vom Typ pAL1 P der Fig. 13 und in den so erhaltenen Vektor vom Typ pAL1N der Fig. 12 später die mutierte C-terminale Hälfte eingeführt, wobei man ebenfalls einen Vektor vom Typ pAL1NC der Fig. 14 erhält.

Mit den oben beschriebenen Expressionsvektoren werden geeignete Bakterien, vorzugsweise Bacillus-Spezies, insbesondere Bacillus subtilis, licheniformis und alcalophilus, transformiert. Die Transformanten werden anschließend in an sich bekannter Weise kultiviert und die gebildete hochalkalische Protease aus dem Kulturmedium isoliert. Die Expressionsvektoren können hierzu sowohl in Bakterien, die noch zur Bildung von Eigenprotease befähigt sind, als auch in Protease-defiziente Bakterien (die keine Eigenprotease mehr bilden) transformiert werden. Bei Wirtsorganismen mit Eigenproteasebildung kann die erfindungsgemäße hochalkalische Protease gewünschtenfalls durch anschließende Reinigungsoperationen, z.B. durch Hochauflösende Flüssigchromatographie (HPLC), von den gebildeten Eigenproteasen befreit werden. Ein solcher Reinigungschritt kann demgegenüber bei Protease-defizienten Wirtsorganismen entfallen, da diese nur (oder im wesentlichen nur) die erfindungsgemäße Protease zu bilden vermögen.

Die folgende Offenbarung gibt zur weiteren Erläuterung der Erfindung typische beispielhafte Ausgestaltungen der Erfindung wieder, ohne jedoch dadurch die Erfindung zu beschränken.

Um die Beispiele zu vereinfachen werden einige häufig wiederkehrende Methoden und Begriffe im folgenden näher erläutert und dann in den einzelnen Beispielen nur noch durch eine Kurzbezeichnung referiert. Sofern nicht anders angegeben, wurde generell nach Methoden gearbeitet, wie sie in Maniatis et al. (Maniatis et al. = T. Maniatis, E. F. Fritsch, J. Sambrook, Molecular Cloning, A Laboratory Manual, Cold Spring Harbor Laboratory, 1982) beschrieben sind.

Hier verwendete Ausgangsvektoren sind käuflich und auf unbeschränkter Basis verfügbar; oder sie können nach an sich bekannten Methoden aus verfügbaren Vektoren hergestellt werden.

Die verschiedenen benutzten Restriktionsendonukleasen gehören zum Stand der Technik und sind kommerziell verfügbar. Die bei Verwendung dieser bekannten Restriktionsendonukleasen jeweils erforderlichen Reaktions-, Kofaktor- und übrigen Bedinungen sind ebenfalls bekannt. Z.B. kann für eine Menge von etwa 1 µg eines Vektors oder eines DNA-Fragments eine Einheit (= 1 U ≙ unit) der Restriktionsendonuklease in etwa 20 µl einer Pufferlösung eingesetzt werden. Ausreichende Inkubationszeiten von etwa einer Stunde bei 37 °C wurden gewöhnlich eingehalten, die Inkubationsbedingungen können aber den gegebenen Erfordernissen angepaßt werden. Nach Inkubation mit einer Restriktionsendonuklease wurde das Protein durch Extraktion (z.B. mit Phenol und Chloroform) entfernt und die geschnittene DNA (z.B. aus der wäßrigen Fraktion durch Fällung mit Ethanol) isoliert und der weiteren Verwendung zugeführt.

An das Schneiden von Vektoren mit Restriktionsendonukleasen kann sich-gegebenenfalls eine Hydrolyse des terminalen 5'-Phosphatrestes mit einer alkalischen Phosphatase (Dephosphorylierung) anschließen. Dadurch kann verhindert werden, daß die beim Schneiden entstandenen Enden der restringierten DNA oder des restringierten Vektors mit sich selbst ligieren und somit die gewünschte Insertion eines Fremd-DNA-Fragmentes in die Restriktionsstelle verhindert würde. Sofern in den Beispielen eine Dephosphorylierung des 5'-Endes vorgenommen wurde, geschah dieses in an sich bekannter Weise. Weitere Angaben zur Durchführung einer Dephosphorylierung und zu dafür benötigten Reagentien können Maniatis et al. (S. 133 - 134) entnommen werden.

Partielle Hydrolyse bedeutet unvollständige Verdauung von DNA durch eine Restriktionsendonuklease. Die Reaktionsbedingungen werden dabei so gewählt, daß in einem DNA-Substrat zwar an einigen, nicht aber an allen Erkennungsstellen für die eingesetzte Restriktionsendonuklease geschnitten wird.

Zur Gewinnung und Isolierung von bestimmten DNA-Fragmenten, z.B. nach Behandlung von DNA mit Restriktionsendonukleasen, wurden die angefallenen DNA-Fragemente in an sich bekannter Weise durch Gelelektrophorese (z.B. auf Agarosegel) getrennt, nachfolgend über das Molekulargewicht (Bestimmung durch Vergleich mit Referenz-DNA-Fragmenten mit bekanntem Molekulargewicht) identifiziert und die gewünschten DNA-Fragmente aus den entsprechenden Gelzonen abgetrennt.

Behandlung mit dem Klenow-Fragment der DNA-Polymerase I aus E. coli bedeutet ein Verfahren zum Auffüllen der inneren 3'-Enden von doppelsträngiger DNA mit Nukleotiden, die zu den Nukleotiden der jeweiligen überstehenden 5'-Enden des DNA-Doppelstranges komplementär sind. Dieses Verfahren wird z.B. verwendet, wenn innere DNA-Strangenden, die aus einer Spaltung von doppelsträngiger DNA mit Restriktionsendonukleasen resultieren, mit Nukleotiden aufgefüllt werden sollen, z.B. um für weitere Ligationen erforderliche glatte DNA-Doppelstrangenden zu erzeugen. Die Behandlung mit dem Klenow-Fragment wird ausgeführt, indem man die geeigneten komplementären Nukleotide mit der aufzufüllenden DNA in Gegenwart einer ausreichenden katalytischen Aktivität des Klenow-Fragments der E. coli-DNA-Polymerase I reagieren läßt (z.B. ca. 15 Min. bei 15 °C). Das Klenow-Fragment sowie weitere für die Klenow-Behandlung benötigte Reagentien sind im Stand der Technik bekannt und kommerziell verfügbar. Weitere Details zur Klenow-Behandlung sind z.B. aus Maniatis et al. (S. 107 bis 108) entnehmbar.

Ligation (ligieren) bedeutet ein Verfahren zur Bildung von Phosphodiesterbindungen zwischen DNA-Fragmenten (siehe z.B. Maniatis et al., S. 146). Ligationen können unter an sich bekannten Bedingungen, z.B. in einem Puffer mit etwa 10 Units T4-DNA-Ligase pro 0,5 µg einer etwa gleich molaren Menge der zu ligierenden DNA-Fragmente, ausgeführt werden.

Unter Transformation wird die Einschleusung von DNA in einen Mikroorganismus verstanden, so daß die DNA in diesem repliziert bzw. exprimiert werden kann. Für die Transformation von E. coli ist z.B. die Calciumchloridmethode nach Mandel et al. (1970, J. Mol. Biol. 53:154) oder nach Maniatis et al. (S.250 bis 251) geeignet. Für Bacillus-Spezies ist z.B. die Methode Anagnostopolous et al. (1961, J. Bact. 81: 791 - 746) geeignet.

Ein Linker ist ein kurzkettiges doppelsträngiges DNA-Fragment, welches einige Erkennungsstellen für Restriktionsendonukleasen aufweist und sich zum Verbindungen von DNA-Fragmenten eignet. Linker werden z.B. beim Rekombinieren von DNA-Fragmenten zu einem Vektor eingesetzt und können zur Einführung bestimmter Erkennungsstellen für Restriktionsendonukleasen in diesen Vektor dienen.

Eine Polyklonierungsstelle (Polylinker) ist ein kurzes bis mittleres doppelsträngiges DNA-Fragment, welches eng benachbart eine Vielzahl von Erkennungsstellen für Restriktionsendonukleasen aufweist. Eine in den Beispielen verwendete, aus dem Vektor M13tg131 entstammende, Polyklonierungsstelle besitzt z.B. eine Größe von etwa 0,07 KB (Kilo Basenpaare) und weist Erkennungsstellen für 14 verschiedene Restriktionsendonukleasen auf.

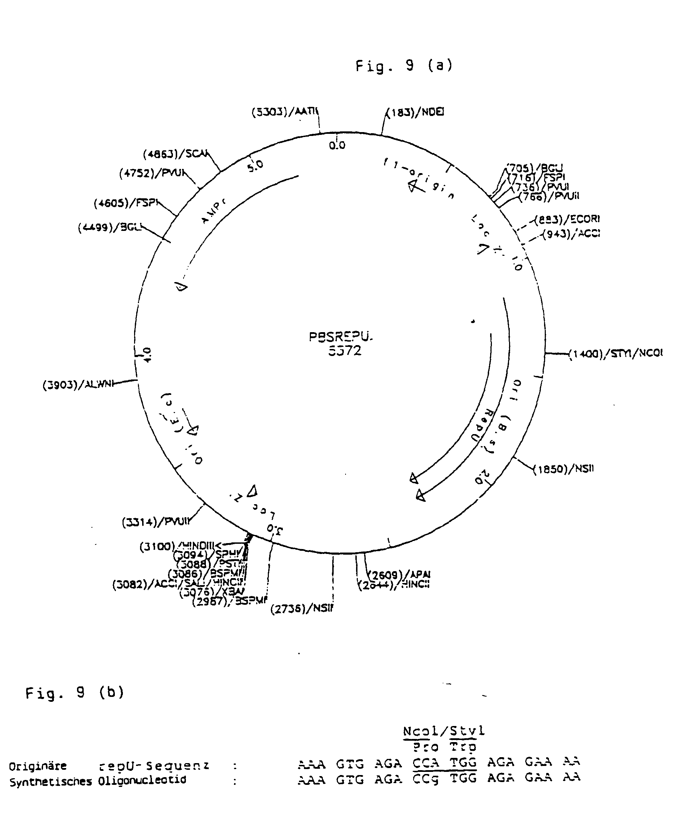

Der im Beispiel 1 eingesetzte und als Bacillus alcalophillus HA1 benannte Bacillus alcalophillus-Stamm ist bei der Deutschen Sammlung von Mikroorganismen (DSM) mit der DSM-Nummer 5466 am 28. Juli 1989 hinterlegt worden. Erläuterungen zu den Figuren:
Figur 1:
   DNA-Sequenz des Aval/Hindlll-Fragmentes mit dem Strukturgen der hochalkalischen Ausgangsprotease aus Bacillus alcalophilus HA1, sowie die Aminosäurensequenz dieser Ausgangsprotease.
Figur 2:
   Restriktionskarte des Plasmids pCLEAN0.
Figur 3:
   Restriktionskarte des Plasmids pCLEAN4.
Figur 4:
   DNA-Sequenzen für die synthetischen Oligonukleotide I - XV und Angabe eliminierter bzw. erzeugter Erkennungsstellen für einzelne Restriktionsendonukleasen; die gegenüber der ur3prünglichen DNA-Sequenz der Ausgangsprotease erzeugten Nukleotidveränderungen sind durch Angabe der veränderten Nukleotide mit kleinen Buchstaben gekennzeichnet.
Figur 5:
   Restriktionskarte des Vektors pCLMUTN1.
Figur 6:
   Restriktionskarte des Vektors pCLMUTC1.
Figur 7:
   Restriktionskarte des Vektors pUB131.
Figur 8:
   Restriktionskarte des Vektors pUBC131.
Figur 9:
   Restriktionskarte des Vektors pBSREPU (Fig. 9a) und synthetische DNA-Sequenz (Fig. 9b) für die Eliminierung der Ncol- bzw. Styl-Erkennungsstelle (die in der DNA-Sequenz enthalten ist, die für das repU-Protein codiert) aus dem Vektor pUBC131.
Figur 10:
   Restriktionskarte des Vektors pUBC132.
Figur 11:
   Restriktionskarte des Plasmids pAL1P.
Figur 12:
   Restriktionskarte des Plasmids pAL1 N.
Figur 13:
   Restriktionskarte des Plasmids pAL1C.
Figur 14:
   Restriktionskarte der Expressionsvektoren vom Typ pAL1 NC für die Expression mutierter und nicht-mutierter hochalkalischer Proteasen.

### Beispiel 1

### Herstellung einer genomischen DNA-Bibliothek aus B. alcalophilus und Isolierung des Gens für die hochalkalische Ausgansprotease

Aus dem Naturisolat Bacillus alcalophilus HA1 (hinterlegt bei der Deutschen Sammlung von Mikroorganismen unter der Nummer DSM 5466) wurde nach der Methode von Saito et al. (1963, Biochim.Biophys.Acta. 72:619-629) chromosomale DNA isoliert und mit der Restriktionsendonuklease Sau3A partiell hydrolisiert. Die Restriktionsfragmente wurden durch Elektrophorese auf einem Agarosegel aufgetrennt und die Fragmente mit einer Größe von 3 bis 8 Kilobasen (KB) wurden isoliert.

Die isolierten und größenselektierten DNA-Fragmente aus Bacillus alcalophilus HA1 wurden mit Vektor-DNA des Plasmids pUB 110 (Herstellung wie in Beispiel 9 beschrieben) in vitro neukombiniert.

Hierzu wurde das Plasmid pUB110 zunächst mit der Restriktionsendonuklease BamHl restringiert und anschließend mit alkalischer Phosphatase aus Kälberdarm dephosphoryliert. Anschließend wurden 2µg der restringierten und dephosphorylierten Vektor-DNA mit 8 pg der B. alcalophilus DNA-Fragmente in einem Gesamtvolumen von 100 µl mit T4-DNA Ligase 24 h bei 16 °C inkubiert.

Mit der erhaltenen in vitro neukombinierten DNA wurden Protoplasten des Stammes Bacillus subtilis BD224 (Bacillus Genetic Stock Center 1 A 46) nach der von S. Chang und N. Cohen (1979, Mol. Gen. Genet. 168: 111 - 115) beschriebenen Methode transformiert. Die Transformanten wurden auf Platten mit Neomycin selektiert und anschließend auf Magermilchagar überführt. Unter 13800 untersuchten Transformanten wurde eine gefunden, die durch Proteolyse des Magermilchagars einen deutlich größeren Hof bildete. Aus diesem Klon wurde die Plasmid-DNA nach Maniatis et al. isoliert. Das in diesem Plasmid enthaltene klonierte Fragment aus der B. alcalophilus-DNA hatte eine Größe von 4,1 KB und enthielt (wie in Beispiel 2 nachgewiesen wurde) die vollständige korrekte DNA-Sequenz für die hochalkalische Protease aus Bacillus alcalophilus HA1. Das Plasmid erhielt die Bezeichnung pCLEAN0. Das Plasmid pCLEAN0 wurde mit verschiedenen Restriktionsendonukleasen geschnitten, die restringierte DNA durch Elektrophorese auf einem Agarosegel auf getrennt und anhand des Bandenmusters eine Restriktionskarte erstellt, die nachträglich anhand des Sequenzierungsergebnisses gemäß Beispiel 4 überprüft wurde. Die Restriktionskarte dieses Plasmids ist in der Fig. 2 wiedergegeben.

### Beispiel 2

### Expression des Strukturgens und Bestimmung der proteolytischen Aktivität der exprimierten Ausgansprotease

Das Plasmid pCLEAN0 wurde erneut in den Stamm B. subtilis BD224 eingebracht und die erhaltenen Transformanten kultiviert. Als Kontrollstämme wurden ein mit dem Plasmid pUB110 transformierter B. subtilis BD224 und der Ausgangsstamm für die Isolierung des Proteasegens, der B. alcalophilus HA1, ebenfalls kultiviert. Der Stamm B. subtilis BD224 (pCLEAN0) und die Kontrollstämme, B. subtilis BD224 (pUB110) und B. alcalophilus HA1 wurden hierzu in einem Medium, das 8 g Nutrient Broth, 40 mg MgSO₄, 0,2 g CaCl₂, 1 mg MnCl₂ und 1 mg FeSO₄ pro Liter enthielt bei 37 °C und 250 Upm inkubiert. Das Medium für die Plasmidhaltigen B. subtilis-Stämme enthielt zusätzlich 10 µg Neomycin/ml. Das Medium für den alcalophilen Ausgangsstamm enthielt pro Liter Medium zusätzlich 10 ml Natriumcarbonatpuffer (1 molar, pH 9,75).

Nach 28 h wurden Proben aus den Kulturen entnommen, zentrifugiert und die proteolytischen Aktivitäten in den überständen bestimmt.

Weiterhin wurden die proteolytischen Aktivitäten auch in Gegenwart des Serinproteasen-Inhibitors PMSF (Phenylmethylsulfonylfluorid) bzw. des Metalloproteasen-Inhibitors EDTA (Ethylendiamintetraessigsäure) bestimmt.

Tabelle 1 zeigt die Ergebnisse in Abwesenheit von Inhibitoren und in Gegenwart der Inhibitoren PMSF bzw. EDTA.

**Tab. 1**

| | Aktivität in Gegenwart von | | |
|---|---|---|---|
| Überstand von | - | PMSF | EDTA |
| B. alcalophilus HA1 | 100% | 1,5% | 95% |
| B. sub. BD224 (pUB110) | 100% | 44% | 51 % |
| B. sub. BD224 (pCLEAN0) | 100 % | 6 % | 78 % |

Für die oben durch Zentrifugieren der Kulturproben erhaltenen Kulturüberstände wurde in Ergänzung der Bestimmung der proteolytischen Aktivitäten, die in diesen überständen enthaltenen Proteine durch isoelektrische Fokussierung aufgetrennt. Diese zeigt, daß der Stamm B. subtilis BD224 (pCLEANO) im Gegensatz zum Kontrollstamm (B. subtilis BD224 mit ausschließlich der Vektor-DNA des Plasmids pUB110) ein Protein ausscheidet, das den gleichen isoelektrischen Punkt aufweist, wie die von B. alcalophilus HA1 produzierte hochalkalische Protease.

Die Bildung von Protease durch den mit pCLEAN0 transformierten B. subtilis BD224 bestätigt, daß die zur Selektion des Proteasestrukturgens im Beispiel 1 benutzten phänomenologischen Eigenschaften, wie Neomycinresistenz und Proteaseaktivität (d.h. verstärkte Hofbildung auf Magermilchagar), an dasselbe Plasmid pCLEAN0 gebunden sind. Ferner zeigen die Ergebnisse, daß das im Plasmid pCLEAN0 enthaltene DNA-Fragment aus B. alcalophilus HA1 die vollständige Information zur Synthese der hochalkalischen B. alcalophilus-Protease enthält, da die durch B. subtilis BD224 (pCLEAN0) gebildete Protease denselben isoelektrischen Punkt wie die ursprüngliche B. alcalophilus-HA1-Protease aufweist und sich zudem auch analog B. alcalophilus-Protease gegenüber Inhibitoren wie PMSF oder EDTA verhält.

### Beispiel 3

### Kontruktion des Plasmids pCLEAN4

Plasmid pCLEAN0 wurde mit den Restriktionsendonukleasen Aval und Hindlll restringiert. Das 2,3 KB große DNA-Fragment wurde isoliert und mit dem Vektor pUB131 (Herstellung wie in Beispiel 10 beschrieben), der zuvor ebenfalls mit Aval und Hindill geschnitten wurde, ligiert.

Das erhaltene Plasmid, das die Bezeichnung pCLEAN4 erhielt, wurde in den Stamm B. subtilis BD224 eingebracht. Die Transformanten waren in der Lage, die hochalkalische Protease auszuscheiden, was zeigt, daß das Aval/HindIII-Fragment das vollständige Strukturgen für die hochalkalische Protease aus B. alcalophilus HA1 enthält. Die Restriktionskarte des Plasmids pCLEAN4 ist in Fig. 3 wiedergegeben.

### Beispiel 4

### Sequenzierung des Strukturgens für die hochalkalische Protease

Zur Herstellung von Einzelstrang-DNA des Proteasestrukturgens wurde das Plasmid pCLEAN4 mit den Restriktionsendonukleasen Aval und Hindlll geschnitten und das etwa 2,3 KB große Aval/Hindlll-DNA-Fragment (Protease-strukturgen) in den Phagemiden pBS (+) oder pBS (-) eingebracht; die Phagemide pBS (+/-) wurden von Stratagene (La Jolla, California) bezogen. Die Nukleotidsequenz des in den isolierten Einzelstrang-Phagemiden enthaltenen Proteasegens wurde nach der Dideoxy-Kettenterminator-Methode von Sanger et al. (1977, Proc. Natl. Acad. Sci. USA 74:5473) und der Methode der basenspezifischen chemischen Spaltung des DNA-Einzelstranges nach Maxam et al. (1980, in Methods in Enzymology, Grossmann L., Moldave K., eds., Academic Press Inc., New York und London, Vol. 65,499) bestimmt. Die ermittelte Nukleotidsequenz und die zugeordnete Aminosäurensequenz der Protease sind in Fig. 1 wiedergegeben. Der Start für die Aminosäurensequenz der reifen hochalkalischen Protease in Position 1190 der Nukleotidsequenz wurde durch Aminosäurensequenzierung des N-terminalen Endes der hochalkalischen Protease bestimmt.

### Beispiel 5

### Herstellung mutierter DNA-Sequenzen durch gerichtete Mutagenese

Die gerichteten Mutationen wurden in DNA-Teilsequenzen des Proteasestrukturgens mit der von Kunkel, T.A. (1985, Proc.Natl.Acad.Sci.USA 82:488-492) beschriebenen "primer extension" Technik durchgeführt. Hierzu wurden die Plasmide pCLMUTN1 (Herstellung wie in Beispiel 6 beschrieben) und pCLMUTC1 (Herstellung wie in Beispiel 7 beschrieben), die zunächst wie nachfolgend beschrieben in ihre uracylierten, einzelträngigen Analoga umgewandelt wurden, eingesetzt. Die Ausgangsvektoren pCLMUTN1 und pCLMUTC1 enthalten nicht die gesamte DNA-Sequenz des Proteasestrukturgens aus B. alcalophilus HA1, sondern nur die N-terminale Hälfte (pCLMUTN1) oder die C-terminale Hälte (pCLMUTC1) desselben.

Diese Vektoren sind als Abkömmlinge eines Phagemiden in gewissem Umfange zur Bildung von Einzelstrang-Vektor-DNA befähigt, die unter den hier gegebenen Bedinungen aus dem zur Replikation dienenden Wirtsorganismus ausgeschleust und isoliert werden konnte.

Jeder dieser Vektoren(wurde nach Maniatis et al. (S. 250 bis 251) mit Hilfe der Cacl₂ Methode in E. coli CJ236 als Wirtsorganismus eingebracht.

Da das Bakterium E. coli CJ236 (Uracil-N-Glycosylase-Mangelmutante) bei der Replikation von Vektoren statt Thymin das Nukleotid Uracil in die DNA-Sequenz des Vektors einbaut, wurden durch Kultivierung der vorstehenden Transformanten die Uracil-haltigen Analoga des Vektors pCLMUTN1 oder pCLMUTC1 erhalten. Diese Uracil-enthaltenden Vektoren sind von den gewöhnlichen Thymin-enthaltenden Vektoren bei in vitro-Reaktionen nicht unterscheidbar. Der Uracil-Gehalt in der Vektor-DNA stört in vitro DNA-Synthesen nicht, da Uracil weder in vitro noch in vivo mutagen ist und Uracil gleichermaßen wie Thymin codiert. Uracylierte Vektoren lassen sich vorteilhaft für die nachfolgenden in vitro Reaktionen der gerichteten Mutagenese einsetzen. Nach Beendigung der Reaktionen kann der Uracil-haltige DNA-Einzelstrang, der als Matrize zur Erzeugung mutierter DNA-Stränge (Vektoren) diente, durch Behandlung mit Uracil-N-Glycosylase beseitigt werden, ohne daß es einer phänotypischen Selektion von Mutanten bedarf. Die Glycosylase-Behandlung kann sowohl mit dem isolierten Enzym als auch mit einem durch Vektor-DNA transformierten E. coli-Stamm mit Uracil-N-Glycosylase-Aktivität durchgeführt werden.

Die für die gerichtete Mutagenese als Matrize benötigte, uracylierte einzelsträngige DNA der Vektoren pCLMUTN1 und pCLMUTC1 wurde hergestellt, indem mit einem der beiden Vektoren transformierte E. coli CJ236-Bakterien kultiviert wurden, die zusätzlich mit dem Helfer-Phagen M13K07 (bezogen von Bio-Rad Laboratories, Richmond, Kalifornien) infiziert wurden.

Der Helfer-Phage selbst ist kaum vermehrungsfähig und zeigt keine störende Interaktion mit der Vektor-DNA der Vektoren pCLMUTN1 oder pCLMUTC1. Seine Aufgabe besteht in der Synthese von Hüllproteinen für die gebildete uracylierte einzelsträngige Vektor-DNA. Umhüllte Einzelstrang-Vektor-DNA wird aus dem Wirtsorganismus E. coli CJ236 ausgeschleust und kann aus dem Kulturmedium isoliert werden. Durch die Mithilfe des Helfer-Phagen wird die qualitative und quantitative Ausbeute an (hier an uracylierter) Einzelstrang-Vektor-DNA wesentlich erhöht.

Die isolierten, uracylierten DNA-Einzelstrang-Vektoren pCLMUTN1 oder pCLMUTC1 wurden mit den nach Beispiel 8 hergestellten, synthetischen Oligonukleotiden hybridisiert, die eine Mutationsstelle enthielten und gleichzeitig als Primer für die nachfolgende Ergänzung zum vollständigen DNA-Doppelstrang mit Mutation dienten.

Die Synthese des zweiten DNA-Stranges wurde unter Zugabe von Nukleotiden mit T4-DNA-Polymerase und die nachfolgende Ligation des neugebildeten Stranges mit T4-DNA-Ligase durchgeführt (Kunkel et al. 1987, Methods in Enzymol. 154, 367 - 382). Die gebildete Doppelstrang-Vektor-DNA wurde in E. coli MC1061 transformiert und die mutierten Vektoren wurden durch überprüfen der entsprechenden unitären Restriktionsendonukleasen-Erkennungsstellen, die mit den synthetischen Oligonukleotiden eingeführt bzw entfernt wurden, identifiziert.

Zur Herstellung von z.B. zwei Mutationen entweder im N-terminalen oder im C-terminalen Teil des Proteasestrukturgens wurde das Verfahren dieses Beispiels nach der Einführung einer ersten Mutation (Verwendung eines ersten synthetischen Oligonukleotids des Beispiels 8) in einen Teil des Proteasestrukturgens in analoger Weise unter Verwendung eines weiteren synthetischen Oligonukleotids des Beispiels 8 zur Einführung einer zweiten Mutation in diesen Teil des Proteasestrukturgens wiederholt. Man erhielt so mutierte Vektoren vom Typ pCLMUTN1 oder pCLMUTC1 mit z.B. zwei Mutationen entweder im N-terminalen oder im C-terminalen Teil des Proteasestrukturgens.

### Beispiel 6

### Konstruktion des Vektors pCLMUTN1

Das in Beispiel 3 hergestellte Plasmid pCLEAN4 wurde mit Aval geschnitten. Die überstehenden Enden ("sticky ends") wurden unter Zugabe der benötigten Nukleotide mit Hilfe des Klenow-Fregments der E. coli DNA-Polymerase I (Maniatis et al., S. 114) zum DNA-Doppelstrang aufgefüllt. Nach anschließender Restriktion dieser DNA mit Xbal wurde das N-terminale 1618 Basenpaar (BP) umfassende Fragment des Proteasegens isoliert und in die Smal/Xbal-Stelle von pBS kloniert. Der resultierende Vektor erhielt die Bezeichnung pCLMUTN1. Die Restriktionskarte dieses Vektors ist in Fig. 5 wiedergegeben.

### Beispiel 7

### Kontruktion des Vektors pCLMUTC1

Das in Beispiel 3 hergestellte Plasmid pCLEAN4 wurde mit den Restriktionsendonukleasen Xbal und Asp718 geschnitten. Das 658 BP umfassende Xbal/Asp718-DoppelstrangDNA-Fragment, welches die C-terminale Hälfte des Protease-strukturgens umfaßt, wurde in die Xbal/Asp718-Stelle von pBS kloniert. Der resultierende Vektor erhielt die Bezeichnung pCLMUTC1. Die Restriktionskarte des Vektors ist in Fig. 6 wiedergegeben.

### Beispiel 8

### Synthese künstlicher Oligonukleotide für die gerichtete Mutagenese

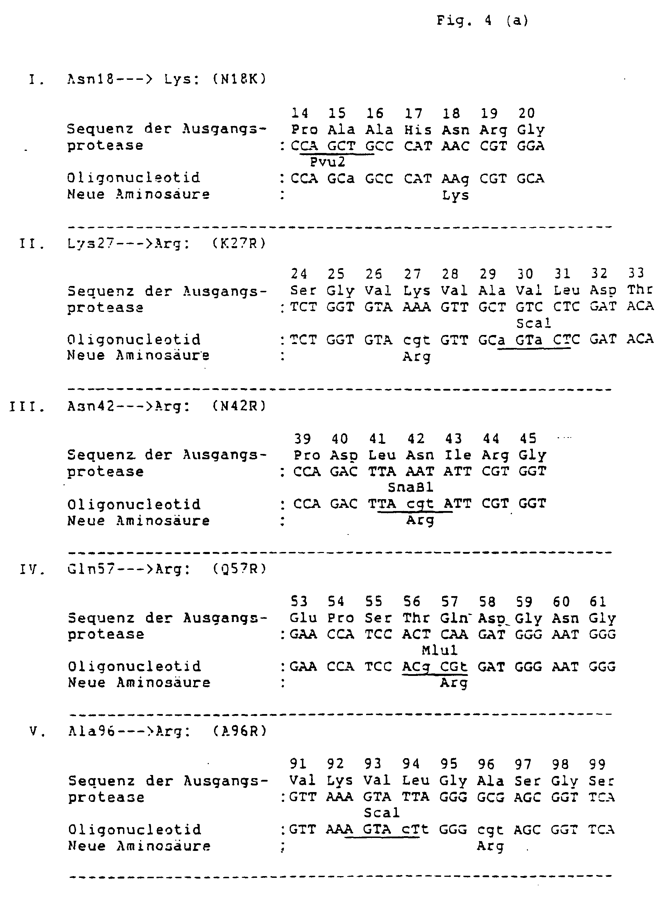

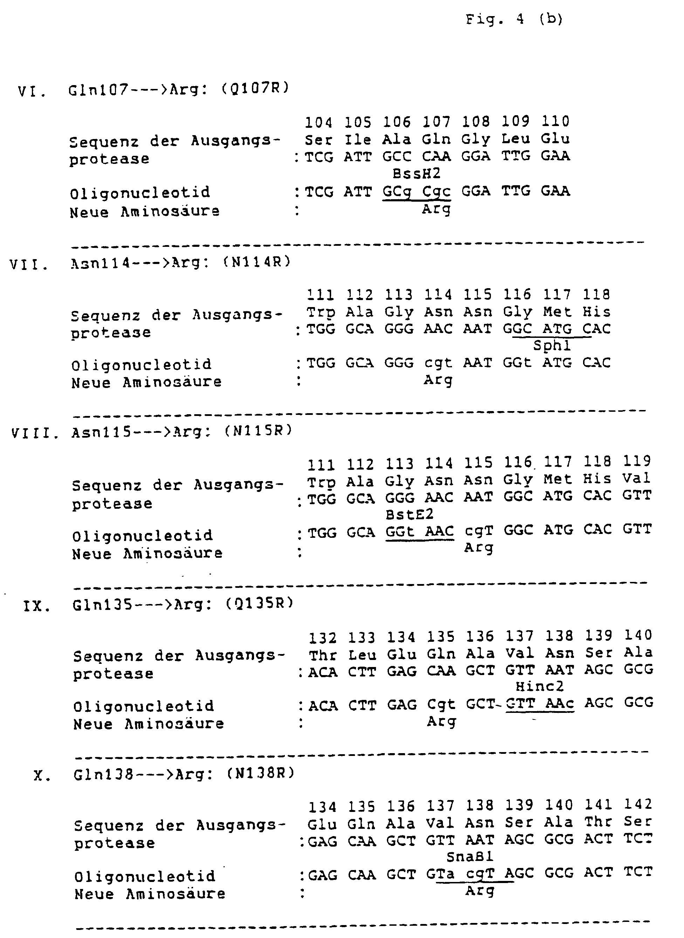

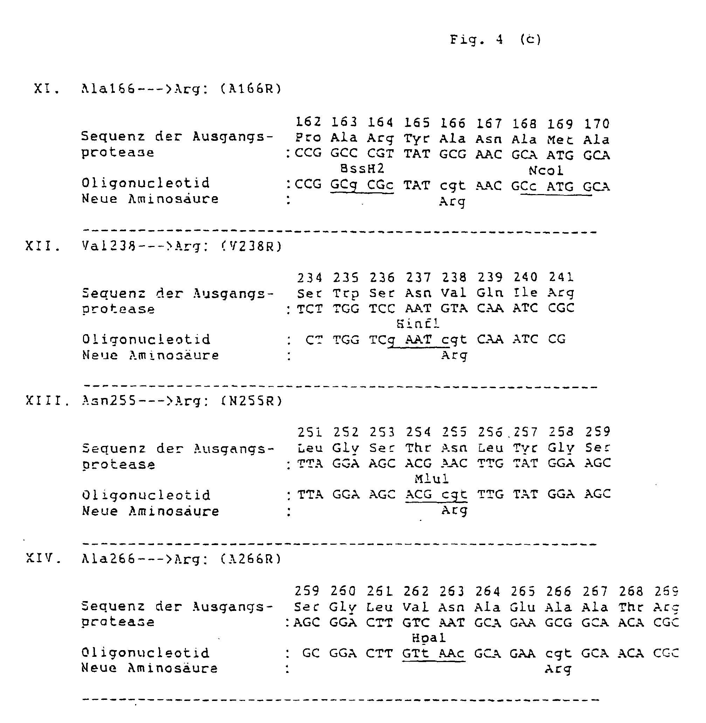

Synthetische Oligonukleotide wurden nach Beaucage S.L. und Caruthers M.H. (1981, Tetrahedron Letters 22: 1859 - 1862) mit β-Cyanoethyl-phosphoramidit in einem Cyclone Synthetiser (Biosearch) hergestellt. Die erhaltenen Oligonukleotide wurden gereinigt durch Elution aus Polyacrylamidgelen und anschließende Entsalzung mit Hilfe von Sephadex-G25-Säulen. Beispiele für die synthetisierten Nukleotidsequenzen und deren Eigenschaften sind in Fig. 4 wiedergegeben. Die Sequenzen der synthetischen Oligonukleotide I bis XIV, die im Verfahren nach Beispiel 5 zur Einführung der Mutationen in das Proteasegen dienten, waren so gewählt, daß sie die nachfolgenden Bedingungen erfüllten.
- Die DNA-Sequenz der synthetischen Oligonukleotide war zur entsprechenden Sequenz des Proteasegens noch soweit komplementär, daß ausreichende Hybridisierungsfähigkeit derselben sichergestellt war.
- Austausch eines oder mehrerer Nukleotide innerhalb des Codons, welches für die auszutauschende Aminosäure codiert, durch andere Nukleotide, so daß dieses mutierte Codon nunmehr für eine stärker basische Aminosäure aus der Gruppe Lysin oder Arginin codierte (Mutationen). Für die neue, stärker basische Aminosäure wurde dasjenige Codon eingesetzt, welches im Proteasegen für die entsprechende, stärker basische Aminosäure am häufigsten vorgefunden wurde.
- Austausch von weiteren Nukleotiden innerhalb anderer Codons, so daß die ursprüngliche Codierung der Aminosäure zwar erhalten blieb, aber dadurch im Proteasegen vorkommende Erkennungssequenzen für Restriktionsendonukleasen entfernt oder neue erzeugt wurden. Diese dienten im Verfahren nach Beispiel 5 zur Erleichterung des Screenings nach den Vektoren mit den mutierten DNA-Sequenzen für die neuen hochalkalischen Proteasen.

### Beispiel 9

### Isolierung und Reinigung des Plasmids pUB110

Aus dem Stamm Bacillus subtilis BD366 (Bacillus Genetic Stock Center 1 E 6) wurde nach der Methode von T.J. Gryczan et al. (1978,J. Bacteriol. 134:318-329) das Plasmid pUB110 isoliert und anschließend nach Maniatis et al. (S. 93) über Cäsiumchlorid-Dichtegradientenzentrifugation gereinigt. Der Vektor pUB110 enthält eine nur einmal vorkommende Restriktionsstelle für die Restriktionsendonuklease BamHl und als Marker eine DNA-Sequenz, die für Antibiotikaresistenz gegenüber Neomycin codiert, sowie für die Replikation in Bacillus-Spezies benötigte DNA-Sequenzen ("origin of replication").

### Beispiel 10

### Konstruktion des Vektors pUB131

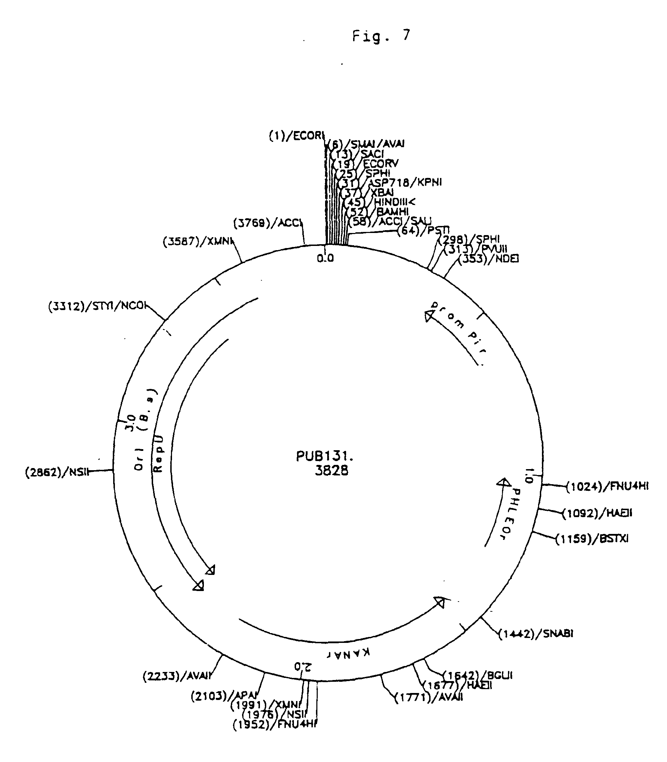

Das nach Beispiel 9 erhaltene Plasmid pUB110 wurde mit EcoRI und BamHI restringiert. Das kleinere DNA-Fragment (790 BP) wurde ersetzt durch einen aus 67 Basenpaaren bestehenden Polylinker, der zuvor als EcoRl/BgIII-Fragment aus dem Vektor M13tg131 (bezogen von Amersham, Buckinghamshire, England) isoliert worden war Der neue Vektor erhielt die Bezeichnung PUB131. Der Vektor pUB131 ist somit ein Abkömmling von pUB110, bei dem das etwa 0,8 KB große EcoRI/BamHI Fragment deletiert und dafür eine Polyklonierungsstelle eingebaut wurde. Die Restriktionskarte dieses Vektors ist in der Fig. 7 wiedergegeben.

### Beispiel 11

### Konstruktion des Vektors pUBC131

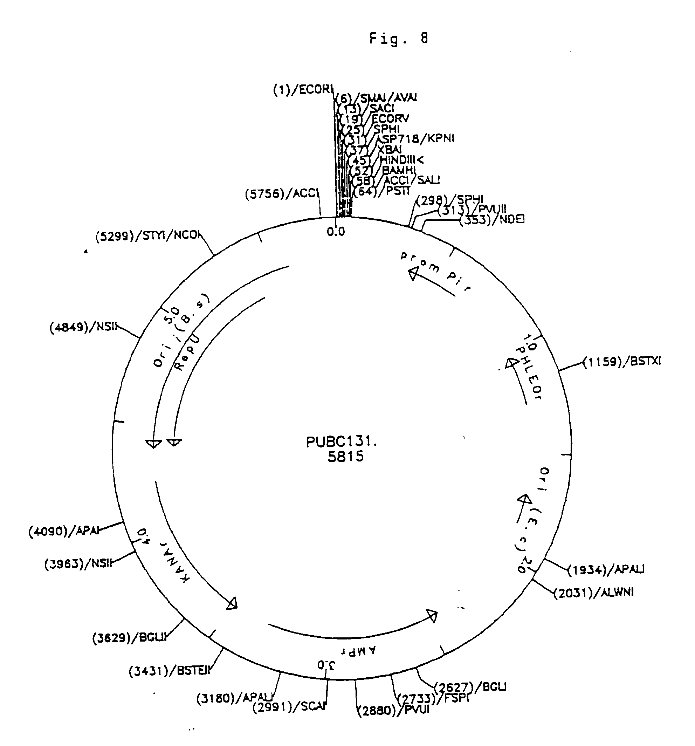

Das Plasmid pUC18 (bezogen von Pharmacia LKB, Uppsala, Schweden) wurde mit Aatll und Pvull geschnitten. Das 1990 Basenpaar große Fragment mit dem β-Lactamase-Gen und dem E. coli "origin of replication" wurde isoliert. Die überstehenden Enden ("sticky ends") wurden unter Zugabe der benötigten Nukleotide mit Hilfe des Klenow-Fragmentes der E. coli DNA-Polymerase I (Maniatis et al., S. 114) zum DNA-Doppelstrang aufgefüllt. Das Fragment wurde anschließend in die SnaBl-Stelle des nach Beispiel 10 erhaltenen Vektors pUB131 eingebaut. Der neue Vektor erhielt die Bezeichnung pUBC131. Die Restriktionskarte dieses Vektors ist in Fig. 8 wiedergegeben.

### Beispiel 12

### Konstruktion des Vektors pUBC132

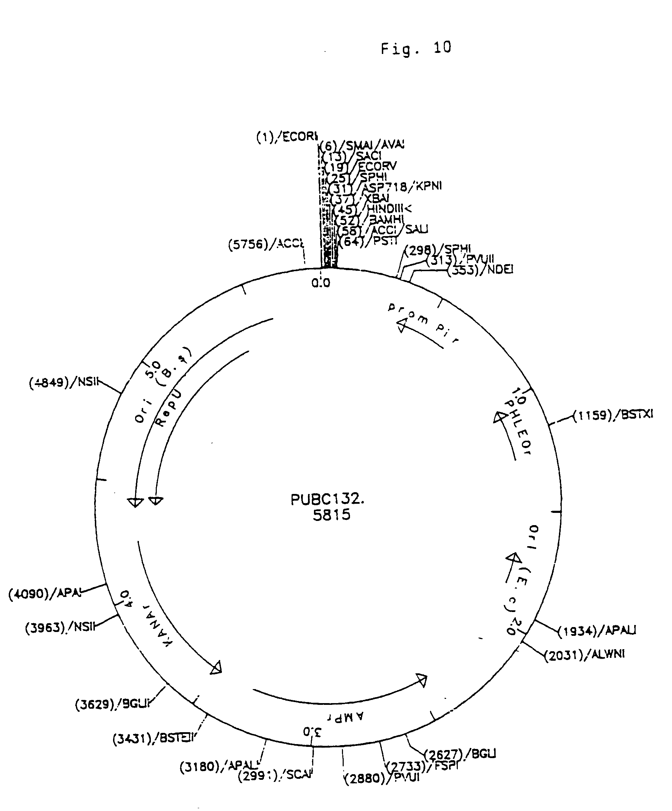

Das 2187 BP EcoRI/Bglll-Fragment des in Beispiel 11 erhaltenen Vektors pUBC131 wurde in die EcoRI/BamHI-Stelle von PBS (+) subkloniert. Der erhaltene Vektor, dessen Restriktionskarte in Fig. 9a wiedergegeben ist, erhielt die Bezeichnung pBSREPU. Anschließend wurde die Ncol- bzw. Styl-Erkennungsstelle, die in der DNA-Sequenz für das repU-Polypeptid im Vektor pBSREPU vorhanden ist (1. Maciag et al. 1988, Mol. Gen. Genet. 212: 232-240), durch gerichtete Mutagenese eliminiert, indem die Nukleotidsequenz CCA TGG durch die Nukleotidsequenz CCG TGG (beide Nukleotidsequenzen codieren für die Aminosäurenfolge Tryptophan-Prolin) ersetzt wurde. Die Durchführung war analog der Verfahrensweise des Beispiels 5. Hierzu wurde uracylierte einzelsträngige DNA des Vektors pBSREPU als Matrize für die gerichtete Mutation zur Eliminierung der Ncol- bzw. Styl-Erkennungsstelle hergestellt. Anschließend wurde diese Matrize analog zur im Beispiel 5 beschriebenen "primer extension"-Technik unter Verwendung des synthetischen Oligonukloetids der Fig. 9b (hergestellt und gereinigt analog zum Verfahren zur Herstellung der synthetischen Oligonukleotide des Beispiels 8) zum DNA-Doppelstrang-Vektor ergänzt und durch Transformation und Kultivierung von E. coli MC 1061 die nunmehr Ncol- bzw. Styl-Erkennungstellen-freien Vektoren isoliert. Das 1726 BP EcoRI/ Apal-Fragment des isolierten Vektors wurde in die EcoRI/Apal-Stelle von pUBC131 eingeführt. Der neue Vektor, dessen Restriktionskarte in Fig. 10 wiedergegeben ist erhielt die Bezeichnung pUBC132.

### Beispiel 13

### Konstruktion des Plasmids pAL1P

Das Plasmid pAL1P wurde hergestellt durch Ligation der folgenden drei Elemente:
- das 2218 Basenpaar große Aval/Ncol-Fragment von pCLEAN4; das Fragment enthält den Promotor und die Prepro-Region der hochalkalischen Ausgangsprotease;
- der nach Beispiel 17 herstellte synthetische Linker; dieser enthält einzelne Erkennungsstellen für die Restriktionsendonukleasen Ncol, Xbal und Asp718, die die Einführung der mutierten N-terminalen bzw. C-terminalen Hälften des Proteasegens aus den mutierten Vektoren pCLMUT1 bzw. pCLMUTC1 oder die Einführung des gesamten Gens der Ausgangsprotease aus dem Plasmid pCLEAN4 ermöglichen;
- das 5776 Basenpaar große Aval/Hindlll-Fragment aus dem in Beispiel 12 hergestellten Vektor pUBC132; dieses Fragment enthält DNA-Sequenzen zur Replikation und selektierbare Marker in E. coli, sowie DNA-Sequenzen zur Replikation und selektierbare Marker in B. subtilis, B. licheniformis und B. alcalophilus.

Die Konstruktion des Vektors pAL1 P wurde in E. coli MC1061 durchgeführt und der Vektor aus Ampicillin-resistenten E. coli-Transformanten isoliert. Die Restriktionskarte des erhaltenen Vektors ist in Fig. 11 wiedergegeben.

### Beispiel 14

### Konstruktion des Plasmids pAL1N

Das Plasmid pAL1N wurde konstruiert, indem zunächst der in Beispiel 1 erhaltene Vektor pCLEAN4 mit den Restriktionsendonukleasen Ncol und Xbal geschnitten und das erhaltene 414 Basenpaar große Ncol/Xbal-Fragment anschließend in die Ncol/Xbal-Stelle des Vektors pAL1P (hergestellt nach Beispiel 13) kloniert wurde. Die Konstruktion des Vektors pAL1N wurde in E. coli MC1061 durchgeführt und der Vektor aus Ampicillin-resistenten E. coli-Transformanten isoliert. Der hergestellte Vektor enthält den N-terminalen Teil der DNA-Sequenz, die für das reife Enzym codiert und die regulatorischen Elemente für die Transkription und Translation der hochalkalischen Protease, sowie die Signal-Sequenz und die Processing-Sequenz. Die Restriktionskarte dieses Vektors ist in Fig. 12 wiedergegeben.

### Beispiel 15

### Konstruktion des Plasmids pAL1C

Das Plasmid pAL1C wurde konstruiert, indem zunächst der in Beispiel 3 erhaltene Vektor pCLEAN4 mit den Restriktionsendonukleasen Xbal und Asp718 geschnitten und das erhaltene 606 Basenpaar große Xbal/Asp718-Fragment in die Xbal/Asp718-Stelle des Vektors pAL1P (hergestellt nach Beispiel 13) kloniert wurde. Die Konstruktion des Vektors pAL1C wurde in E. coli MC1061 durchgeführt und der Vektor aus Ampicillin-resistenten E. coli-Transformanten isoliert. Der hergestellte Vektor enthält den C-terminalen Teil der DNA-Sequenz, die für die reife Protease codiert und die regulatorischen Elemente für die Transkription und Translation der hochalkalischen Protease, sowie die Signal-Sequenz und die Processing-Sequenz. Die Restriktionskarte dieses Vektors ist in Fig. 13 wiedergegeben.

### Beispiel 16

### Kontruktion der Expressionsvektoren pAL1 NC

Es wurden Expressionsvektoren mit Mutationen im C-terminalen Teil der Protease-DNA-Sequenz, Expressionsvektoren mit Mutationen im N-terminalen Teil der Protease-DNA-Sequenz, Expressionsvektoren mit Mutationen im N-und C-terminalen Teil der DNA-Sequenz und zu Vergleichszwecken auch Expressionsvektoren ohne Mutationen in der Protease-DNA-Sequenz hergestellt.
A. Expressionsvektor mit Mutationen im N-terminalen Teil der DNA-Sequenz der Protease
   Der nach Beispiel 5 durch gerichtete Mutagenese erhaltene mutierte Vektor pCLMUTN1 wurde mit den Restriktionsendonukleasen Ncol und Xbal geschnitten. Das isolierte 414 Basenpaar große Ncol/Xbal-Fragment (mutierter N-terminaler Teil des Proteasestrukturgens mit den erfindungsgemäßen Mutationen N18K, K27R, N42R, Q57R, N114R, N115R, Q135R, N138R wurde in die Ncol/Xbal-Stelle des nach Beispiel 15 erhaltenen Plasmides pAL1C kloniert. Der jeweils erhaltene Vektor stellt einen vollständigen Expressionsvektor mit geeignetem Leseraster zur Expression der mutierten Protease dar. Die Vektoren wurden folgendermaßen bezeichnet:
   pALN18K = Expressionsvektor für die Protease mit der Mutation N18K;
   pALK27R = Expressionsvektor für die Protease mit der Mutation K27R;
   pALN42R = Expressionsvektor für die Protease mit der Mutation N42R;
   pALQ57R = Expressionsvektor für die Protease mit der Mutation Q57R;
   pALA96R = Expressionsvektor für die Protease mit der Mutation A96R; (für die weitere Verwendung unter "C.")
   pALQ107R = Expressionsvektor für die Protease mit der Mutation Q107R: (für die weitere Verwendung unter "C.")
   pALN114R = Expressionsvektor für die Protease mit der Mutation N114R;
   pALN115R = Expressionsvektor für die Protease mit der Mutation N115R;
   pALQ135R = Expressionsvektor für die Protease mit der Mutation Q135R;
   pALN138R = Expressionsvektor für die Protease mit der Mutation N138R;
   pAL27/115 = Expressionsvektor für die Protease mit der Mutation K27R/N115R;
   pAL27/135 = Expressionsvektor für die Protease mit der Mutation K27R/Q135R.

   Zusätzlich wurden die nicht erfindungsgemäßen Mutationen A96R und Q107R für die weitere Verwendung unter "C." dieses Beispiels erzeugt (pALA96R, pALQ107R).
B. Expressionsvektor mit Mutationen im C-terminalen Teil der DNA-Sequenz der Protease
   Der nach Beispiel 5 durch gerichtete Mutagenese erhaltene mutierte Vektor pCLMUTC1 wurde mit den Restriktionsendonukleasen Xbal und Asp718 geschnitten. Das isolierte 606 Basenpaar große Xbal/Asp718-Fragment (mutierter C-terminaler Teil des Proteasestrukturgens mit den erfindungsgemäßen Mutationen, V238R, N255R, A266R) wurde in die Xbal/Asp718-Stelle des nach Beispiel 14 erhaltenen Plasmides pAL1N kloniert. Der jeweils erhaltene Vektor stellt einen vollständigen Expressionsvektor mit geeignetem Leseraster zur Expression der mutierten Protease dar. Die Vektoren wurden folgendermaßen bezeichnet:
   pALV238R = Expressionsvektor für die Protease mit der Mutation V238R;
   pALN255R = Expressionsvektor für die Protease mit der Mutation N255R,
   pALA266R = Expressionsvektor für die Protease mit der Mutation A266R.
C. Expressionsvektor mit Mutationen im C-terminalen und N-terminalen Teil der Protease-DNA-Sequenz
   Der in diesem Beispiel unter A. hergestellte Expressionsvektor pALA96R und der unter B. hergestellte Expressionsvektor pALA266R wurden jeweils mit den Restriktionsendonukleasen Xbal und Aval geschnitten. Das 1612 Basenpaar große Fragment des Plasmids pALA96R wurde mit dem 6388 Basenpaar großen Fragment des Plasmids pALA266R ligiert. Das resultierende Plasmid erhielt die Bezeichnung pAL96/266.
   Analog wurde aus dem in diesem Beispiel unter A. hergestellten Expressionsvektor pALQ107R und dem unter B. hergestellten Expressionsvektor pALV238R das Plasmid mit der Bezeichnung pAL107/238 hergestellt.
D. Expressionsvektor mit der nicht-mutierten DNA-Sequenz der Ausgangsprotease
   Der Expressionsvektor mit dem nicht-mutierten Ausgangsstrukturgen der Protease wurde erhalten, indem entweder das 414 Basenpaar große, nicht-mutierte Ncol/Xbal-Fragment aus dem nach Beispiel 3 erhaltenen Plasmid pCLEAN4 in die Ncol/Xbal-Stelle des nach Beispiel 15 erhaltenen Plasmides pAL1C kloniert wurde; oder indem das 606 Basenpaar große Xbal/Asp718-Fragment aus dem nach Beispiel 3 erhaltenen Plasmid pCLEAN4 in die Xbal/ Asp718-Stelle des nach Beispiel 14 erhaltenen Plasmides pAL1N kloniert wurde. Die so erhaltenen Vektoren sind vollständige Expressionsvektoren mit geeignetem Leseraster zur Expression der nicht-mutierten hochalkalischen Ausgangsprotease.
   Die Konstruktionen der vier vorstehend aufgeführten Expressionsvektoren wurde jeweils in E. coli MC1061 durchgeführt. Aus Ampicillin-resistenten E. coli-Transformanten wurden die Expressionsvektoren isoliert. Zur Expression mutierter und nicht-mutierter Proteasegene Wurden die in diesem Beispiel hergestellten und isolierten Expressionsvektoren in B. subtilits BD224 eingebracht. Die Selektion erfolgte hier nach Neomycin- oder Phleomycin-Resistenz. Die Transformanten waren zur Produktion der mutierten (Transformanten mit Vektoren aus A., B. und C.) bzw. der nicht-mutierten (Transformanten mit Vektor aus D.) hochalkalischen Protease befähigt.
   Die Restriktionskarte dieser Vektoren des Typs pAL1NC ist in Fig. 14 wiedergegeben.

### Beispiel 17

### Synthese eines DNA-Doppelstrang-Linkers

Zur Synthese eines doppelsträngigen Linkers mit überstehenden 5'-Enden, welcher sich durch eine DNA-Sequenz auszeichnet, die für die, in der angegebenen Reihenfolge eng benachbart oder direkt aufeinander abfolgenden Erkennungsstellen für die Restriktionsendonukleasen Ncol am Beginn, gefolgt von Xbal und Asp718 im Verlaufe und HindIII am Ende der Sequenz codiert, wurden zunächst die beiden Einzelstrang-DNA-Sequenzen analog zur Synthese der synthetischen Oligonukleotide in Beispiel 8 jeweils für sich hergestellt und gereinigt. Die erhaltenen DNA-Einzelstränge wurden nachfolgend miteinander zum Doppelstrang hybridisiert. Der hergestellte synthetische DNA-Doppelstrang-Linker mit folgender Sequenz und folgenden einzelnen Erkennungsstellen für Restriktionsendonukleasen 
5' - CCATGGTCTAGAGGTACCA - 3'
3' - CAGATCTCCATGGTTCGAA - 5'
^ ^ ^ ^
NcoI XbaI Asp718
HindIII
 wurde für die Konstruktion des Vektors nach Beispiel 13 eingesetzt.

### Beispiel 18

### Herstellung der mutierten hochalkalischen Proteasen und zu Vergleichszwecken auch der Ausgangsprotease

50 ml Vorkulturmedium (20 g Tryptone, 10 g Hefe-Extrakt, 5 g NaCI, 75 g lösliche Stärke, 10 ml Maisquellwasser pro Liter) wurden mit einer Kolonie der zu testenden Stämme (jeweils mit einem der nach Beispiel 16 hergestellten Vektoren pAL1 NC transformierte B. subtilis BD224) beimpft. Die Kultur wurde für 16 h bei 37 °C und 250 Upm inkubiert. Mit 2,5 ml dieser Kultur wurden 50 ml Hauptkulturmedium (30 g Sojamehl, 90 g Kartoffelstärke, 10 g Na-Caseinat und 10 ml Maisquellwasser pro Liter) beimpft. Die Hauptkultur wurde unter den gleichen Bedingungen wie die Vorkultur inkubiert. Nach 72 h wurden die Kulturen zentrifugiert.

Aus den überständen wurde die hochalkalische Protease über HPLC (Ultropac Column Typ TSK-CM-2SW von LKB; Elutionspuffer 0,05 M Na-Acetat mit pH 6 und 0,8 M Na-Acetat mit pH 6) gereinigt.

Im folgenden wurden die Wascheigenschaften der optimierten (mutierten) Proteasen im Vergleich zur (nicht-mutierten) Ausgangsprotease bestimmt.

### Beispiel 19

Die Waschtests wurden in einem Linitest mit EMPA 117 (11 cm x 11 cm; Polyester/Baumwolle-Mischgewebe, verschmutzt mit Milch, Blut und Tusche) als Testgewebe (bezogen von der Eidgenössischen Materialprüfungsanstalt, Sankt Gallen, Schweiz) durchgeführt. Gewaschen wurde mit 6 g/l lEC-Testwaschmittel mit Perborat, Type 1 (Lever Sunlicht GmbH, Mannheim; Zusammensetzung: 6,4 Gew.-% Linerare Alkylsulfonate, 2,3 Gew.-% Ethoxylierte Fettalkohole mit 14 % Ethoxygruppen, 2,8 Gew.-% Natriumseife, 35 Gew.-% Natriumtripolyphosphat, 6 Gew.-% Natriumsilikat, 1,5 Gew-% Magnesiumsilikat, 1 Gew.-% Carboxymethylcellulose, 0,2 Gew.-% Ethylendiamintetraessigsäure (EDTA), 0,2 Gew.-% optische Aufheller (Stilbentyp), 16,8 Gew.-% Natriumsulfat und 7,8 Gew.-% Wasser als sprühgetrocknetes Pulver ohne Bleichaktivator sowie 20 Gew.-% Natriumperborat-Tetrahydrat) in Wasser mit 15° dH. Der pH-Wert der Wachflotte betrug pH 10,5. Die Waschdauer betrug 30 Minuten bei 45 °C und es wurden jeweils 20 U Protease (hergestellt wie in Beispiel 18 beschrieben) pro 100 ml eingesetzt. Nach Beendigung des Waschvorganges wurden die Stoffstücke dreimal mit Leitungswasser gespült, getrocknet und anschließend gebügelt. Die Reflektion der Testlappen wurde auf beiden Seiten je viermal bestimmt. Tabelle 2 gibt jeweils die Durchschnittswerte von 16 Waschversuchen wieder.

**Tab. 2**

| Protease | Reflektion | Δ |
|---|---|---|
| Blindwert (ohne Protease) | 48,8 % | - |
| Ausgangsprotease | 71,1% | 100% |
| K27R | 72,8% | 107,6% |
| N42R | 71,9% | 103,6% |
| Q57R | 72,3% | 105,4% |
| N115R | 71,7% | 102,7% |
| K27R/N115R | 74,1% | 113,5% |
| Q107R/V238R | 73% | 108,5% |

### Beispiel 20

Die Waschversuche wurden analog zum Beispiel 19 durchgeführt. Als Waschpulver wurde ein Anion- und Niotensid. Natriumtripolyphosphat als Komplexbildner, Natriumsilikat, Natriumperborat als Bleichmittel und Natriumsulfat als Stellmittel enthaltendes Waschmittel in einer für Europa geeigneten Formulierung verwendet. Das Waschergebnis ist in Tabelle 3 wiedergegeben.

**Tab 3**

| Protease | Reflektion | Δ |
|---|---|---|
| Blindwert (ohne Protease) | 47,4 % | - |
| Ausgangsprotease | 65,6 % | 100 % |
| N18 K | 66,2 % | 103,3 % |
| K27R | 66,4% | 104,4 % |
| N114R | 66,5% | 104,9% |
| Q135R | 66,3% | 103,8 % |
| N138R | 67,3% | 109,3% |
| V238R | 66 % | 102,2% |
| N255R | 65,9% | 101,6 % |
| K27R/Q135R | 67,4% | 109,9% |
| A96R/A266R | 66,6 % | 105,5 % |

## Patentansprüche (Patentansprüche für folgende(n) Vertragsstaat(en): AT, BE, CH, DE, DK, FR, GB, IT, LI, LU, NL, SE)

1. Hochalkalische Protease, dadurch gekennzeichnet, daß sie ein pH-Optimum von 10 bis 12,5 und ein Molekulargewicht von 26000 bis 28000 g/mol besitzt, und daß sie eine Aminosäuresequenz aufweist, welche wenigstens 80% Homologie zu der in Fig. 1 angegebenen Aminosäuresequenz besitzt und sich von dieser
- in mindestens einer der Positionen 18, 57, 114, 115, 135, 188, 189, 238, 255 der Fig. 1, vorzugsweise 18, 57, 114, 115, 135, 238, 255, oder in einer der dazu homologen Positionen dadurch unterscheidet, daß die in der betreffenden Position befindliche Aminosäure durch die stärker basische Aminosäure Lysin oder Arginin ausgetauscht ist, oder
- in Position 266 oder in einer dazu homologen Position dadurch unterscheidet, daß die in der betreffenden Position befindliche Aminosäure durch die Aminosäure Lysin ausgetauscht ist.

2. Hochalkalische Protease nach Anspruch 1, dadurch gekennzeichnet, daß sie eine Aminosäuresequenz aufweist, in welcher die Aminosäure in 1 bis 3, vorzugsweise in 1 oder 2, der im Anspruch 1 angegebenen Positionen durch eine stärker basische Aminosäure ausgetauscht ist.

3. Hochalkalische Protease nach einem der vorhergehenden Ansprüche, dadurch gekennzeichnet, daß sie eine Aminosäuresequenz aufweist, welche mindestens 90%, vorzugsweise mindestens 95%, Homologie zu der in Fig. 1 angegebenen Aminosäuresequenz aufweist.

4. Hochalkalische Protease nach einem der vorhergehenden Ansprüche, dadurch gekennzeichnet, daß sie eine Aminosäuresequenz aufweist, in welcher die Aminosäure in der Position 18 gegen Lysin ausgetauscht ist.

5. Hochalkalische Protease nach einem der Ansprüche 1 bis 3, dadurch gekennzeichnet, daß sie eine Aminosäuresequenz aufweist, in welcher die Aminosäure in mindestens einer der Positionen 57, 114, 115, 135, 238 oder 255 gegen Arginin ausgetauscht ist.

6. DNA-Sequenz, codierend eine hochalkalische Protease gemäß Anspruch 1 mit einer Aminosäuresequenz, welche wenigstens 80% Homologie zu der in Fig. 1 angegebenen Aminosäuresequenz aufweist und sich von dieser
- in mindestens einer der Positionen 18, 57, 114, 115, 135, 188, 189, 238, 255 der Fig. 1, vorzugsweise 18, 57, 114, 115, 135, 238, 255, oder in einer der dazu homologen Positionen dadurch unterscheidet, daß die in der betreffenden Position befindliche Aminosäure durch die stärker basische Aminosäure Lysin oder Arginin ausgetauscht ist, oder
- in Position 266 oder in einer dazu homologen Position dadurch unterscheidet, daß die in der betreffenden Position befindliche Aminosäure durch die Aminosäure Lysin ausgetauscht ist.

7. DNA-Sequenz nach Anspruch 6, dadurch gekennzeichnet, daß sie eine Aminosäuresequenz codiert, in welcher die Aminosäure in 1 bis 3, vorzugsweise in 1 oder 2, der im Anspruch 6 angegebenen Positionen durch eine stärker basische Aminosäure ausgetauscht ist.

8. DNA-Sequenz nach Anspruch 7, dadurch gekennzeichnet, daß sie eine Aminosäuresequenz codiert, welche mindestens 90%, vorzugsweise mindestens 95% Homologie zu der in Fig. 1 angegebenen Aminosäuresequenz aufweist.

9. DNA-Sequenz nach einem der Ansprüche 6 bis 8, dadurch gekennzeichnet, daß sie eine Aminosäuresequenz codiert, in welcher die Aminosäure in der Position 18 gegen Lysin ausgetauscht ist.

10. DNA-Sequenz nach einem der Ansprüche 6 bis 9, dadurch gekennzeichnet, daß sie eine Aminosäuresequenz codiert, in welcher die Aminosäure in mindestens einer der Positionen 57, 114, 115, 135, 238 oder 255 gegen Arginin ausgetauscht ist.

11. Waschmittel, enthaltend eine hochalkalische Protease gemäß einem der Ansprüche 1 bis 5.

12. Waschmittel nach Anspruch 11, enthaltend übliche Formulierungsbestandteile für Waschmittel, wie Tenside, Builder und Bleichmittel, sowie gegebenenfalls weitere als Waschmittelbestandteile übliche Zusatz- und Hilfsstoffe.

13. Verfahren zur Herstellung einer optimierten hochalkalischen Protease gemäß einem der Ansprüche 1 bis 5, dadurch gekennzeichnet, daß man einen transformierten Mikroorganismus, der eine DNA-Sequenz nach einem der Ansprüche 6 bis 10 enthält, kultiviert und aus dem Kulturmedium die optimierte hochalkalische Protease, die die in den Ansprüchen 6 bis 10 genannte (n) Aminosäuresubstitution(en) aufweist, isoliert.

## Patentansprüche (Patentansprüche für folgende(n) Vertragsstaat(en): GR)

1. Hochalkalische Protease, dadurch gekennzeichnet, daß sie ein pH-Optimum von 10 bis 12,5 und ein Molekulargewicht von 26000 bis 28000 g/mol besitzt, und daß sie eine Aminosäurensequenz aufweist, welche wenigstens 80 % Homologie zu der in Fig. 1 angegebenen Aminosäurensequenz besitzt und sich von dieser in mindestens einer der Positionen 18, 27, 42, 57, 114, 115, 135, 138, 188, 189, 238, 255, 266 der Fig. 1, vorzugsweise 18, 27 42, 57, 114, 115, 135, 138, 238, 255, 266, oder in einer der dazu homologen Positionen dadurch unterscheidet, daß die in der betreffenden Position befindliche Aminosäure durch die stärker basische Aminosäure Lysin oder Arginin ausgetauscht ist.

2. Hochalkalische Protease nach Anspruch 1, dadurch gekennzeichnet, daß sie eine Aminosäurensequenz aufweist, in welcher die Aminosäure in 1 bis 3, vorzugsweise in 1 oder 2, der im Anspruch 1 angegebenen Positionen durch eine stärker basische Aminosäure ausgetauscht ist.

3. Hochalkalische Protease nach einem der vorhergehenden Ansprüche, dadurch gekennzeichnet, daß sie eine Aminosäurensequenz aufweist, welche mindestens 90 %, vorzugsweise mindestens 95 %, Homologie zu der in Fig. 1 angegebenen Aminosäurensequenz besitzt.

4. Hochalkalische Protease nach einem der vorhergehenden Ansprüche, dadurch gekennzeichnet, daß sie eine Aminoäurensequenz aufweist, in welcher die Aminosäure in der Position 18 gegen Lysin ausgetauscht ist.

5. Hochalkalische Protease nach einem der Ansprüche 1 bis 3, dadurch gekennzeichnet, daß sie eine Aminoäurensequenz aufweist, in welcher die Aminosäure in mindestens einer der Positionen 27, 42, 57, 114, 115, 135, 138, 238, 255 oder 266 gegen Arginin ausgetauscht ist.

6. DNA-Sequenz, codierend für eine hochalkalische Protease gemäß Anspruch 1 mit einer Aminosäurensequenz, welche wenigstens 80 % Homologie zu der in Fig. 1 angegebenen Aminosäurensequenz besitzt und sich von dieser in mindestens einer der Positionen 18, 27, 42, 57, 114, 115, 135, 138, 188, 189, 238, 255, 266 der Fig. 1, vorzugsweise 18, 27, 42, 57, 114, 115, 135, 138, 238, 255, 266, oder in einer der dazu homologen Positionen dadurch unterscheidet, daß die in der betreffenden Position befindliche Aminosäure durch die stärker basische Aminosäure Lysin oder Arginin ausgetauscht ist.

7. DNA-Sequenz nach Anspruch 6, dadurch gekennzeichnet, daß sie für eine Aminosäurensequenz codiert, in welcher die Aminosäure in 1 bis 3, vorzugsweise in 1 oder 2, der im Anspruch 6 angegebenen Positionen durch eine stärker basische Aminosäure ausgetauscht ist.

8. DNA-Sequenz nach Anspruch 7, dadurch gekennzeichnet, daß sie für eine Aminosäurensequenz codiert, welche mindestens 90 %, vorzugsweise mindestens 95 %, Homologie zu der in Fig. 1 angegebenen Aminosäurensequenz besitzt.

9. DNA-Sequenz nach einem der Ansprüche 6 bis 8, dadurch gekennzeichnet, daß sie für eine Aminosäurensequenz codiert, in welcher die Aminosäure in der Position 18 gegen Lysin ausgetauscht ist.

10. DNA-Sequenz nach einem der Ansprüche 6 bis 9, dadurch gekennzeichnet, daß sie für eine Aminosäurensequenz codiert, in welcher die Aminosäure in mindestens einer der Positionen 27, 42, 57, 114, 115, 135, 138, 238, 255 oder 266 gegen Arginin ausgetauscht ist.

11. Waschmittel, enthaltend eine hochalkalische Protease gemäß einem der Ansprüche 1 bis 5.

12. Waschmittel nach Anspruch 11, enthaltend übliche Formulierungsbestandteile für Waschmittel wie Tenside, Builder und Bleichmittel, sowie gegebenenfalls weitere als Waschmittelbestandteile übliche Zusatz- und Hilfsstoffe.

13. Verfahren zur Herstellung einer optimierten hochalkalischen Protease gemäß Anspruch 1, dadurch gekennzeichnet, daß man einen transformierten Mikroorganismus,
der eine DNA-Sequenz enthält, die für eine Aminosäurensequenz codiert, welche wenigstens 80 % Homologie zu der in Fig. 1 angegebenen Aminosäurensequenz besitzt und sich von dieser in mindestens einer der Positionen 18, 27, 42, 57, 114, 115, 135, 138, 188, 189, 238, 255, 266 der Fig. 1, vorzugsweise 18, 27, 42, 57, 114, 115, 135, 138, 238, 255, 266, oder in einer der dazu homologen Positionen dadurch unterscheidet, daß die in der betreffenden Position befindliche Aminosäure gegen die stärker basische Aminosäure Lysin oder Arginin ausgetauscht ist,
kultiviert und aus dem Kulturmedium die optimierte hochalkalische Protease, bei der in mindestens einer der vorgenannten Positionen die dort befindliche Aminosäure gegen die stärker basische Aminosäure Lysin oder Arginin ausgetauscht ist, isoliert.

## Patentansprüche (Patentansprüche für folgende(n) Vertragsstaat(en): ES)

1. Verfahren zur Herstellung einer hochalkalischen Protease, dadurch gekennzeichnet, daß man eine optimierte hochalkalische Protease mit einem pH-Optimum von 10 bis 12,5 und einem Molekulargewicht von 26000 bis 28000 g/mol herstellt, indem man einen transformierten Mikroorganismus,
der eine DNA-Sequenz enthält, die eine Aminosäuresequenz codiert, welche wenigstens 80% Homologie zu der in Fig. 1 angegebenen Aminosäuresequenz besitzt und sich von dieser
- in mindestens einer der Positionen 18, 57, 114, 115, 135, 188, 189, 238, 255 der Fig. 1, vorzugsweise 18, 57, 114, 115, 135, 238, 255, oder in einer der dazu homologen Positionen dadurch unterscheidet, daß die in der betreffenden Position befindliche Aminosäure durch die stärker basische Aminosäure Lysin oder Arginin ausgetauscht ist, oder
- in Position 266 oder in einer dazu homologen Position dadurch unterscheidet, daß die in der betreffenden Position befindliche Aminosäure durch die Aminosäure Lysin ausgetauscht ist,
kultiviert und aus dem Kulturmedium die optimierte hochalkalische Protease, bei der in mindestens einer der vorgenannten Positionen die dort befindliche Aminosäure gegen die stärker basische Aminosäure Lysin oder Arginin ausgetauscht ist, isoliert.

2. Verfahren zur Herstellung einer hochalkalischen Protease nach Anspruch 1, dadurch gekennzeichnet, daß sie eine Aminosäuresequenz aufweist, in welcher die Aminosäure in 1 bis 3, vorzugsweise in 1 oder 2, der im Anspruch 1 angegebenen Positionen durch eine stärker basische Aminosäure ausgetauscht ist.

3. Verfahren zur Herstellung einer hochalkalischen Protease nach einem der vorhergehenden Ansprüche, dadurch gekennzeichnet, daß sie eine Aminosäuresequenz aufweist, welche mindestens 90%, vorzugsweise mindestens 95%, Homologie zu der in Fig. 1 angegebenen Aminosäuresequenz aufweist.

4. Verfahren zur Herstellung einer hochalkalischen Protease nach einem der vorhergehenden Ansprüche, dadurch gekennzeichnet, daß sie eine Aminosäuresequenz aufweist, in welcher die Aminosäure in der Position 18 gegen Lysin ausgetauscht ist.

5. Verfahren zur Herstellung einer hochalkalischen Protease nach einem der Ansprüche 1 bis 4, dadurch gekennzeichnet, daß sie eine Aminosäuresequenz aufweist, in welcher die Aminosäure in mindestens einer der Positionen 57, 114, 115, 135, 238 oder 255 gegen Arginin ausgetauscht ist.

6. Verfahren zur Herstellung einer DNA-Sequenz,
codierend eine hochalkalische Protease mit einem pH-Optimum von 10 bis 12,5 und einem Molekulargewicht von 26000 bis 28000 g/mol und mit einer Aminosäuresequenz, welche wenigstens 80% Homologie zu der in Fig. 1 angegebenen Aminosäuresequenz aufweist und sich von dieser
- in mindestens einer der Positionen 18, 57, 114, 115, 135, 188, 189, 238, 255 der Fig. 1, vorzugsweise 18, 57, 114, 115, 135, 238, 255, oder in einer der dazu homologen Positionen dadurch unterscheidet, daß die in der betreffenden Position befindliche Aminosäure durch die stärker basische Aminosäure Lysin oder Arginin ausgetauscht ist; oder
- in Position 266 oder in einer dazu homologen Position dadurch unterscheidet, daß die in der betreffenden Position befindliche Aminosäure durch die Aminosäure Lysin ausgetauscht ist,
durch Kultivierung eines Bacillus-Stammes, der eine Protease mit wenigstens 80% Homologie zu der in Fig. 1 angegebenen Aminosäuresequenz produziert, Isolierung der die Ursprungsprotease codierenden DNA-Sequenz und Erzeugung von Punktmutationen in dieser DNA-Sequenz, so daß die mutierte DNA-Sequenz nun eine hochalkalische Protease codiert, die sich mindestens in einer der gegenüber der Ursprungsprotease vorstehend angegebenen Aminosäureaustausche unterscheidet, Erzeugung eines Expressionsvektors mit einer derart mutierten DNA-Sequenz und Transformation eines geeigneten Mikroorganismus, sowie Isolierung der die mutierte hochalkalische Protease codierenden DNA-Sequenz.

7. Verfahren zur Herstellung einer DNA-Sequenz nach Anspruch 6, dadurch gekennzeichnet, daß sie eine Aminosäuresequenz codiert, in welcher die Aminosäure in 1 bis 3, vorzugsweise in 1 oder 2, der im Anspruch 6 angegebenen Positionen durch eine stärker basische Aminosäure ausgetauscht ist.

8. Verfahren zur Herstellung einer DNA-Sequenz nach Anspruch 7, dadurch gekennzeichnet, daß sie eine Aminosäuresequenz codiert, welche mindestens 90%, vorzugsweise mindestens 95% Homologie zu der in Fig. 1 angegebenen Aminosäuresequenz aufweist.

9. Verfahren zur Herstellung einer DNA-Sequenz nach einem der Ansprüche 6 bis 8, dadurch gekennzeichnet, daß sie eine Aminosäuresequenz codiert, in welcher die Aminosäure in der Position 18 gegen Lysin ausgetauscht ist.

10. Verfahren zur Herstellung einer DNA-Sequenz nach einem der Ansprüche 6 bis 9, dadurch gekennzeichnet, daß sie eine Aminosäuresequenz codiert, in welcher die Aminosäure in mindestens einer der Positionen 57, 114, 115, 135, 238 oder 255 gegen Arginin ausgetauscht ist.

11. Waschmittel, enthaltend eine gemäß einem der Ansprüche 1 bis 5 hergestellte, hochalkalische Protease.

12. Waschmittel nach Anspruch 11, enthaltend übliche Formulierungsbestandteile für Waschmittel, wie Tenside, Builder und Bleichmittel, sowie gegebenenfalls weitere als Waschmittelbestandteile übliche Zusatz- und Hilfsstoffe.

## Claims (Claims for the following Contracting State(s): AT, BE, CH, DE, DK, FR, GB, IT, LI, LU, NL, SE)

1. Highly alkaline protease, characterised in that it has a pH optimum of 10 to 12.5 and a molecular weight of 26000 to 28000 g/mol, and that it has an amino-acid sequence which has at least 80% homology with the amino-acid sequence indicated in Fig. 1 and differs from the latter
- in at least one of the positions 18, 57, 114, 115, 135, 188, 189, 238, 255 in Fig. 1, preferably 18, 57, 114, 115, 135, 238, 255, or in one of the positions homologous thereto, in that the amino acid located in the relevant position has been replaced by the more strongly basic amino acid lysine or arginine, or
- in position 266, or in one of the positions homologous thereto, in that the amino acid located in the relevant position has been replaced by the amino acid lysine.

2. Highly alkaline protease according to Claim 1, characterised in that it has an amino-acid sequence in which the amino acid in 1 to 3, preferably in 1 or 2, of the positions indicated in Claim 1 has been replaced by a more strongly basic amino acid.

3. Highly alkaline protease according to one of the preceding claims, characterised in that it has an amino-acid sequence which has at least 90%, preferably at least 95%, homology with the amino-acid sequence indicated in Fig. 1.

4. Highly alkaline protease according to one of the preceding claims, characterised in that it has an amino-acid sequence in which the amino acid in the position 18 has been replaced by lysine.

5. Highly alkaline protease according to one of Claims 1 to 3, characterised in that it has an amino-acid sequence in which the amino acid in at least one of the positions 57, 114, 115, 135, 238 or 255 has been replaced by arginine.

6. DNA sequence coding for a highly alkaline protease according to Claim 1 with an amino-acid sequence which has at least 80% homology with the amino-acid sequence indicated in Fig. 1 and differs from the latter
- in at least one of the positions 18, 57, 114, 115, 135, 188, 189, 238, 255 in Fig. 1, preferablyl8, 57, 114, 115, 135, 238, 255, or in one of the positions homologous thereto, in that the amino acid located in the relevant position has been replaced by the more strongly basic amino acid lysine or arginine, or
- in position 266, or in one of the positions homologous thereto, in that the amino acid located in the relevant position has been replaced by the amino acid lysine.

7. DNA sequence according to Claim 6, characterised in that it codes for an amino-acid sequence in which the amino acid in 1 to 3, preferably in 1 or 2, of the positions indicated in Claim 6 has been replaced by a more strongly basic amino acid.

8. DNA sequence according to Claim 7, characterised in that it codes for an amino-acid sequence which has at least 90%, preferably at least 95%, homology to the amino-acid sequence indicated in Fig. 1.

9. DNA sequence according to one of Claims 6 to 8, characterised in that it codes for an amino-acid sequence in which the amino acid in the position 18 has been replaced by lysine.

10. DNA sequence according to one of Claims 6 to 9, characterised in that it codes for an amino-acid sequence in which the amino acid in at least one of the positions 57, 114, 115, 135, 238 or 255 has been replaced by arginine.

11. Detergent, containing a highly alkaline protease according to one of Claims 1 to 5.

12. Detergent according to Claim 11, containing conventional ingredients for formulating detergents, such as surfactants, builders and bleaching agents, and, where appropriate, other additives and auxiliaries customary as ingredients for detergents.

13. Process for the preparation of an optimised highly alkaline protease according to one of Claims 1 to 5, characterised in that a transformed microorganism which contains a DNA sequence in accordance with one of Claims 6 to 10 is cultivated, and the optimised highly alkaline protease which has the amino acid substitution(s) referred to in Claims 6 to 10 is isolated from the culture medium.

## Claims (Claims for the following Contracting State(s): GR)

1. Highly alkaline protease, characterised in that it has a pH optimum of 10 to 12.5 and a molecular weight of 26000 to 28000 g/mol, and that it has an amino-acid sequence which has at least 80% homology with the amino-acid sequence indicated in Fig. 1 and differs from the latter in at least one of the positions 18, 27, 42, 57, 114, 115, 135, 138, 188, 189, 238, 255, 266 in Fig. 1, preferably 18, 27, 42, 57, 114, 115, 135, 138, 238, 255, 266, or in one of the positions homologous thereto, in that the amino acid located in the relevant position has been replaced by the more strongly basic amino acid lysine or arginine.

2. Highly alkaline protease according to Claim 1, characterised in that it has an amino-acid sequence in which the amino acid in 1 to 3, preferably in 1 or 2, of the positions indicated in Claim 1 has been replaced by a more strongly basic amino acid.

3. Highly alkaline protease according to one of the preceding claims, characterised in that it has an amino-acid sequence which has at least 90%, preferably at least 95%, homology with the amino-acid sequence indicated in Fig. 1.

4. Highly alkaline protease according to one of the preceding claims, characterised in that it has an amino-acid sequence in which the amino acid in the position 18 has been replaced by lysine.

5. Highly alkaline protease according to one of Claims 1 to 3, characterised in that it has an amino-acid sequence in which the amino acid in at least one of the positions 27, 42, 57, 114, 115, 135, 138, 238, 255 or 266 has been replaced by arginine.

6. DNA sequence coding for a highly alkaline protease according to Claim 1 with an amino-acid sequence which has at least 80% homology with the amino-acid sequence indicated in Fig. 1 and differs from the latter in at least one of the positions 18, 27, 42, 57, 114, 115, 135, 138, 188, 189, 238, 255, 266 in Fig. 1, preferably 18, 27, 42, 57, 114, 115, 135, 138, 238, 255, 266, or in one of the positions homologous thereto, in that the amino acid located in the relevant position has been replaced by the more strongly basic amino acid lysine or arginine.

7. DNA sequence according to Claim 6, characterised in that it codes for an amino-acid sequence in which the amino acid in 1 to 3, preferably in 1 or 2, of the positions indicated in Claim 6 has been replaced by a more strongly basic amino acid.

8. DNA sequence according to Claim 7, characterised in that it codes for an amino-acid sequence which has at least 90%, preferably at least 95%, homology to the amino-acid sequence indicated in Fig. 1.

9. DNA sequence according to one of Claims 6 to 8, characterised in that it codes for an amino-acid sequence in which the amino acid in the position 18 has been replaced by lysine.

10. DNA sequence according to one of Claims 6 to 9, characterised in that it codes for an amino-acid sequence in which the amino acid in at least one of the positions 27, 42, 57, 114, 115, 135, 138, 238, 255 or 266 has been replaced by arginine.

11. Detergent, containing a highly alkaline protease according to one of Claims 1 to 5.

12. Detergent according to Claim 11, containing conventional ingredients for formulating detergents, such as surfactants, builders and bleaching agents, and, where appropriate, other additives and auxiliaries customary as ingredients for detergents.

13. Process for the preparation of an optimised highly alkaline protease according to Claim 1, characterised in that a transformed microorganism which contains a DNA sequence which codes for an amino-acid sequence which has at least 80% homology with the amino-acid sequence indicated in Fig. 1 and which differs from the latter in at least one of the positions 18, 27, 42, 57, 114, 115, 135, 138, 188, 189, 238, 255, 266 in Fig. 1, preferably 18, 27, 42, 57, 114, 115, 135, 138, 238, 255, 266, or in one of the positions homologous thereto, in that the amino acid located in the relevant position has been replaced by the more strongly basic amino acid lysine or arginine,
is cultivated, and the optimised highly alkaline protease in which in at least one of the above-mentioned positions the amino acid located there is replaced by the more strongly basic amino acid lysine or arginine, is isolated from the culture medium.

## Claims (Claims for the following Contracting State(s): ES)

1. Process for the preparation of a highly alkaline protease, characterised in that an optimised highly alkaline protease with a pH optimum of 10 to 12.5 and a molecular weight of 26000 to 28000 g/mol is produced in that a transformed microorganism
which contains a DNA sequence which codes for an amino-acid sequence which has at least 80% homology with the amino-acid sequence indicated in Fig. 1 and which differs from the latter
- in at least one of the positions 18, 57, 114, 115, 135, 188, 189, 238, 255 in Fig. 1, preferably 18, 57, 114, 115, 135, 238, 255, or in one of the positions homologous thereto, in that the amino acid located in the relevant position has been replaced by the more strongly basic amino acid lysine or arginine, or
- in position 266, or in one of the positions homologous thereto, in that the amino acid located in the relevant position has been replaced by the amino acid lysine,
is cultivated, and the optimised highly alkaline protease in which in at least one of the above-mentioned positions the amino acid located there is replaced by the more strongly basic amino acid lysine or arginine, is isolated from the culture medium.

2. Process for the preparation of a highly alkaline protease according to Claim 1, characterised in that it has an amino-acid sequence in which the amino acid in 1 to 3, preferably in 1 or 2, of the positions indicated in Claim 1 has been replaced by a more strongly basic amino acid.

3. Process for the preparation of a highly alkaline protease according to one of the preceding claims, characterised in that it has an amino-acid sequence which has at least 90%, preferably at least 95%, homology with the amino-acid sequence indicated in Fig. 1.

4. Process for the preparation of a highly alkaline protease according to one of the preceding claims, characterised in that it has an amino-acid sequence in which the amino acid in the position 18 has been replaced by lysine.

5. Process for the preparation of a highly alkaline protease according to one of Claims 1 to 4, characterised in that it has an amino-acid sequence in which the amino acid in at least one of the positions 57, 114, 115, 135, 238 or 255 has been replaced by arginine.

6. Process for the preparation of a DNA sequence
coding for a highly alkaline protease with a pH optimum of 10 to 12.5 and a molecular weight of 26000 to 28000 g/mol and with an amino-acid sequence which has at least 80% homology with the amino-acid sequence indicated in Fig. 1 and differs from the latter
- in at least one of the positions 18, 57, 114, 115, 135, 188, 189, 238, 255 in Fig. 1, preferably 18, 57, 114, 115, 135, 238, 255, or in one of the positions homologous thereto, in that the amino acid located in the relevant position has been replaced by the more strongly basic amino acid lysine or arginine, or
- in position 266, or in one of the positions homologous thereto, in that the amino acid located in the relevant position has been replaced by the amino acid lysine.
by cultivation of a bacillus strain which produces a protease which has at least 80% homology with the amino-acid sequence indicated in Fig. 1, isolation of the DNA sequence coding for the original protease and production of point mutations in this DNA sequence, so that the mutated DNA sequence then codes for a highly alkaline protease which differs at least in one of the amino-acid exchanges indicated above relative to the original protease, production of an expression vector with a DNA sequence thus mutated, and transformation of a suitable microorganism, and isolation of the DNA sequence coding for the mutated highly alkaline protease.

7. Process for the preparation of a DNA sequence according to Claim 6, characterised in that it codes for an amino-acid sequence in which the amino acid in 1 to 3, preferably in 1 or 2, of the positions indicated in Claim 6 has been replaced by a more strongly basic amino acid.

8. Process for the preparation of a DNA sequence according to Claim 7, characterised in that it codes for an amino-acid sequence which has at least 90%, preferably at least 95%, homology to the amino-acid sequence indicated in Fig. 1.

9. Process for the preparation of a DNA sequence according to one of Claims 6 to 8, characterised in that it codes for an amino-acid sequence in which the amino acid in the position 18 has been replaced by lysine.

10. Process for the preparation of a DNA sequence according to one of Claims 6 to 9, characterised in that it codes for an amino-acid sequence in which the amino acid in at least one of the positions 57, 114, 115, 135, 238 or 255 has been replaced by arginine.

11. Detergent, containing a highly alkaline protease prepared according to one of Claims 1 to 5.

12. Detergent according to Claim 11, containing conventional ingredients for formulating detergents, such as surfactants, builders and bleaching agents, and, where appropriate, other additives and auxiliaries customary as ingredients for detergents.

## Revendications (Revendications pour l'(les) Etat(s) contractant(s) suivant(s): AT, BE, CH, DE, DK, FR, GB, IT, LI, LU, NL, SE)

1. Protéase fortement alcaline, caractérisée en ce qu'elle a un optimum de pH de 10 à 12,5 et une masse moléculaire de 26 000 à 28 000 g/mol, et en ce qu'elle présente une séquence d'acides aminés qui possède une homologie d'au moins 80 % avec la séquence d'acides aminés indiquée sur la Figure 1, et qui se distingue de cette dernière
- sur au moins l'une des positions 18, 57, 114, 115, 135, 188, 189, 238, 255 de la Figure 1, de préférence 18, 57, 114, 115, 135, 238, 255, ou sur l'une des positions qui leur sont homologues, par le fait que l'acide aminé se trouvant sur la position considérée est remplacé par l'acide aminé plus fortement basique lysine ou arginine, ou
- sur la position 266, ou sur une position qui lui est homologue, par le fait que l'acide aminé se trouvant sur la position considérée est remplacé par l'acide aminé lysine.

2. Protéase fortement alcaline selon la revendication 1, caractérisée en ce qu'elle présente une séquence d'acides aminés dans laquelle l'acide aminé sur 1 à 3, de préférence 1 ou 2, des positions indiquées dans la revendication 1, est remplacé par un acide aminé plus fortement basique.

3. Protéase fortement alcaline selon l'une des revendications précédentes, caractérisée en ce qu'elle présente une séquence d'acides aminés qui présente une homologie d'au moins 90 % et de préférence d'au moins 95 % avec la séquence d'acides aminés indiquée sur la Figure 1.

4. Protéase fortement alcaline selon l'une des revendications précédentes, caractérisée en ce qu'elle présente une séquence d'acides aminés dans laquelle l'acide aminé sur la position 18 est remplacé par la lysine.

5. Protéase fortement alcaline selon l'une des revendications 1 à 3, caractérisée en ce qu'elle présente une séquence d'acides aminés dans laquelle l'acide aminé sur au moins l'une des positions 57, 114, 115, 135, 238 ou 255 est remplacé par l'arginine.

6. Séquence d'ADN codant pour une protéase hautement alcaline selon la revendication 1, ayant une séquence d'acides aminés qui présente une homologie d'au moins 80 % avec la séquence d'acides aminés indiquée sur la Figure 1, et qui se distingue de cette dernière
- sur au moins l'une des positions 18, 57, 114, 115, 135, 188, 189, 238, 255 de la Figure 1, de préférence 18, 57, 114, 115, 135, 238, 255, ou sur l'une des positions qui leur sont homologues, par le fait que l'acide aminé se trouvant sur la position considérée est remplacé par l'acide aminé plus fortement basique lysine ou arginine, ou
- sur la position 266, ou sur une position qui lui est homologue, par le fait que l'acide aminé se trouvant sur la position considérée est remplacé par l'acide aminé lysine.

7. Séquence d'ADN selon la revendication 6, caractérisée en ce qu'elle code pour une séquence d'acides aminés dans laquelle l'acide aminé est, sur 1 à 3 et de préférence 1 ou 2 des positions indiquées dans la revendication 6, remplacé par un acide aminé plus fortement basique.

8. Séquence d'ADN selon la revendication 7, caractérisée en ce qu'elle code pour une séquence d'acides aminés qui présente une homologie d'au moins 90 % et de préférence d'au moins 95 % avec la séquence d'acides aminés indiquée sur la Figure 1.

9. Séquence d'ADN selon l'une des revendications 6 à 8, caractérisée en ce qu'elle code pour une séquence d'acides aminés dans laquelle l'acide aminé sur la position 18 est remplacé par la lysine.

10. Séquence d'ADN selon l'une des revendications 6 à 9, caractérisée en ce qu'elle code pour une séquence d'acides aminés dans laquelle l'acide aminé est, au moins sur l'une des positions 57, 114, 115, 135, 238 ou 255, remplacé par l'arginine.

11. Détergent contenant une protéase fortement alcaline selon l'une des revendications 1 à 5.

12. Détergent selon la revendication 11, contenant des constituants usuels de formulation pour détergents, tels que des tensioactifs, des adjuvants et des agents de blanchiment, et éventuellement d'autres additifs et adjuvants usuels en tant que constituants de détergents.

13. Procédé de fabrication d'une protéase fortement alcaline optimisée selon l'une des revendications 1 à 5, caractérisé en ce qu'on cultive un micro-organisme transformé, qui contient une séquence d'ADN selon l'une des revendications 6 à 10, et qu'on isole du milieu de culture la protéase fortement alcaline optimisée, qui présente la ou les substitutions d'acides aminés mentionnées dans les revendications 6 à 10.

## Revendications (Revendications pour l'(les) Etat(s) contractant(s) suivant(s): GR)

1. Protéase fortement alcaline, caractérisée en ce qu'elle a un optimum de pH de 10 à 12,5 et une masse moléculaire de 26 000 à 28 000 g/mol, et en ce qu'elle présente une séquence d'acides aminés qui possède une homologie d'au moins 80 % avec la séquence d'acides aminés indiquée sur la Figure 1, et qui se distingue de cette dernière sur au moins l'une des positions 18, 27, 42, 57, 114, 115, 135, 188, 189, 238, 255, 266, de la Figure 1, de préférence 18, 27, 42, 57, 114, 115, 135, 238, 255, 266, ou sur l'une des positions qui leur sont homologues, par le fait que l'acide aminé se trouvant sur la position considérée est remplacé par l'acide aminé plus fortement basique lysine ou arginine.

2. Protéase fortement alcaline selon la revendication 1, caractérisée en ce qu'elle présente une séquence d'acides aminés dans laquelle l'acide aminé sur 1 à 3, de préférence 1 ou 2, des positions indiquées dans la revendication 1, est remplacé par un acide aminé plus fortement basique.

3. Protéase fortement alcaline selon l'une des revendications précédentes, caractérisée en ce qu'elle présente une séquence d'acides aminés qui présente une homologie d'au moins 90 % et de préférence d'au moins 95 % avec la séquence d'acides aminés indiquée sur la Figure 1.

4. Protéase fortement alcaline selon l'une des revendications précédentes, caractérisée en ce qu'elle présente une séquence d'acides aminés dans laquelle l'acide aminé sur la position 18 est remplacé par la lysine.

5. Protéase fortement alcaline selon l'une des revendications 1 à 3, caractérisée en ce qu'elle présente une séquence d'acides aminés dans laquelle l'acide aminé sur au moins l'une des positions 57, 114, 115, 135, 238 ou 255 est remplacé par l'arginine.

6. Séquence d'ADN codant pour une protéase hautement alcaline selon la revendication 1, ayant une séquence d'acides aminés qui possède une homologie d'au moins 80 % avec la séquence d'acides aminés indiquée sur la Figure 1, et qui se distingue de cette dernière sur au moins l'une des positions 18, 27, 42, 57, 114, 115, 135, 188, 189, 238, 255, 266, de la Figure 1, de préférence 18, 27, 42, 57, 114, 115, 135, 238, 255, 266, ou sur l'une des positions qui leur sont homologues, par le fait que l'acide aminé se trouvant sur la position considérée est remplacé par l'acide aminé plus fortement basique lysine ou arginine

7. Séquence d'ADN selon la revendication 6, caractérisée en ce qu'elle code pour une séquence d'acides aminés dans laquelle l'acide aminé est, sur 1 à 3 et de préférence 1 ou 2 des positions indiquées dans la revendication 6, remplacé par un acide aminé plus fortement basique.

8. Séquence d'ADN selon la revendication 7, caractérisée en ce qu'elle code pour une séquence d'acides aminés qui présente une homologie d'au moins 90 % et de préférence d'au moins 95 % avec la séquence d'acides aminés indiquée sur la Figure 1.

9. Séquence d'ADN selon l'une des revendications 6 à 8, caractérisée en ce qu'elle code pour une séquence d'acides aminés dans laquelle l'acide aminé sur la position 18 est remplacé par la lysine.

10. Séquence d'ADN selon l'une des revendications 6 à 9, caractérisée en ce qu'elle code pour une séquence d'acides aminés dans laquelle l'acide aminé est, au moins sur l'une des positions 27, 42, 57, 114, 115, 135, 238, 255 ou 266 remplacé par l'arginine.

11. Détergent contenant une protéase fortement alcaline selon l'une des revendications 1 à 5.

12. Détergent selon la revendication 11, contenant des constituants usuels de formulation pour détergents, tels que des tensioactifs, des adjuvants et des agents de blanchiment, et éventuellement d'autres additifs et adjuvants usuels en tant que constituants de détergents.

13. Procédé de fabrication d'une protéase fortement alcaline optimisée selon la revendications 1, caractérisé en ce qu'on cultive un micro-organisme transformé, qui contient une séquence d'ADN qui code pour une séquence d'acides aminés qui possède une homologie d'au moins 80 % avec la séquence d'acides aminés indiquée sur la Figure 1, et qui se distingue de cette dernière sur au moins l'une des positions 18, 27, 42, 57, 114, 115, 135, 188, 189, 238, 255, 266, de la Figure 1, de préférence 18, 27, 42, 57, 114, 115, 135, 238, 255, 266, ou sur l'une des positions qui leur sont homologues, par le fait que l'acide aminé se trouvant sur la position considérée est remplacé par l'acide aminé plus fortement basique lysine ou arginine,
et qu'on isole du milieu de culture la protéase fortement alcaline optimisée, dans laquelle, sur au moins l'une des positions mentionnées ci-dessus, l'acide aminé qui s'y trouve est remplacé par l'acide aminé plus fortement basique lysine ou arginine.

## Revendications (Revendications pour l'(les) Etat(s) contractant(s) suivant(s): ES)

1. Procédé de fabrication d'une protéase fortement alcaline, caractérisé en ce qu'on prépare une protéase hautement alcaline optimisée ayant un optimum de pH de 10 à 12,5 et une masse moléculaire de 26 000 à 28 000 g/mol, par le fait que l'on cultive un micro-organisme transformé qui contient une séquence d'ADN codant pour une séquence d'acides aminés qui présente une homologie d'au moins 80 % avec la séquence d'acides aminés indiquée sur la Figure 1, et qui se distingue de cette dernière
- sur au moins l'une des positions 18, 57, 114, 115, 135, 188, 189, 238, 255 de la Figure 1, de préférence 18, 57, 114, 115, 135, 238, 255, ou sur l'une des positions qui leur sont homologues, par le fait que l'acide aminé se trouvant sur la position considérée est remplacé par l'acide aminé plus fortement basique lysine ou arginine, ou
- sur la position 266, ou sur une position qui lui est homologue, par le fait que l'acide aminé se trouvant sur la position considérée est remplacé par l'acide aminé lysine,
et que l'on isole du milieu de culture la protéase fortement alcaline optimisée, dans laquelle, sur au moins. l'une des positions mentionnées, l'acide aminé qui s'y trouve est remplacé par l'acide aminé plus fortement basique lysine ou arginine.

2. Procédé de préparation d'une protéase fortement alcaline selon la revendication 1, caractérisée en ce qu'elle présente une séquence d'acides aminés dans laquelle l'acide aminé sur 1 à 3, de préférence 1 ou 2, des positions indiquées dans la revendication 1, est remplacé par un acide aminé plus fortement basique.

3. Procédé de préparation d'une protéase fortement alcaline selon l'une des revendications précédentes, caractérisée en ce qu'elle présente une séquence d'acides aminés qui présente une homologie d'au moins 90 % et de préférence d'au moins 95 % avec la séquence d'acides aminés indiquée sur la Figure 1.

4. Procédé de préparation d'une protéase fortement alcaline selon l'une des revendications précédentes, caractérisée en ce qu'elle présente une séquence d'acides aminés dans laquelle l'acide aminé sur la position 18 est remplacé par la lysine.

5. Procédé de préparation d'une protéase fortement alcaline selon l'une des revendications 1 à 4, caractérisée en ce qu'elle présente une séquence d'acides aminés dans laquelle l'acide aminé sur au moins l'une des positions 57, 114, 115, 135, 238 ou 255 est remplacé par l'arginine.

6. Procédé de préparation d'une séquence d'ADN codant pour une protéase fortement alcaline ayant un optimum de pH de 10 à 12,5 et une masse moléculaire de 26 000 à 28 000 g/mol, et ayant une séquence d'acides aminés qui présente une homologie d'au moins 80 % avec la séquence d'acides aminés indiquée sur la Figure 1, et qui se distingue de cette dernière
- sur au moins l'une des positions 18, 57, 114, 115, 135, 188, 189, 238, 255 de la Figure 1, de préférence 18, 57, 114, 115, 135, 238, 255, ou sur l'une des positions qui leur sont homologues, par le fait que l'acide aminé se trouvant sur la position considérée est remplacé par l'acide aminé plus fortement basique lysine ou arginine, ou
- sur la position 266, ou sur une position qui lui est homologue, par le fait que l'acide aminé se trouvant sur la position considérée est remplacé par l'acide aminé lysine,
par culture d'une souche de Bacillus qui produit une protéase ayant une homologie d'au moins 80 % avec la séquence d'acides aminés indiquée sur la Figure 1, isolement de la séquence d'ADN codant pour la protéase initiale, et production de mutations ponctuelles dans cette séquence d'ADN, de façon que la séquence d'ADN mutée code maintenant pour une protéase fortement alcaline, qui se distingue par au moins l'un des remplacements d'acides aminés indiqués ci-dessus par rapport à la protéase initiale, production d'un vecteur d'expression ayant une séquence d'ADN mutée de cette manière, et transformation d'un micro-organisme approprié, et isolement de la séquence d'ADN codant pour la protéase fortement alcaline mutée.

7. Procédé de préparation d'une séquence d'ADN selon la revendication 6, caractérisée en ce qu'elle code pour une séquence d'acides aminés dans laquelle l'acide aminé est, sur 1 à 3 et de préférence 1 ou 2 des positions indiquées dans la revendication 6, remplacé par un acide aminé plus fortement basique.

8. Procédé de préparation d'une séquence d'ADN selon la revendication 7, caractérisée en ce qu'elle code pour une séquence d'acides aminés qui présente une homologie d'au moins 90 % et de préférence d'au moins 95 % avec la séquence d'acides aminés indiquée sur la Figure 1.

9. Procédé de préparation d'une séquence d'ADN selon l'une des revendications 6 à 8, caractérisée en ce qu'elle code pour une séquence d'acides aminés dans laquelle l'acide aminé sur la position 18 est remplacé par la lysine.

10. Procédé de préparation d'une séquence d'ADN selon l'une des revendications 6 à 9, caractérisée en ce qu'elle code pour une séquence d'acides aminés dans laquelle l'acide aminé est, au moins sur l'une des positions 57, 114, 115, 135, 238 ou 255, remplacé par l'arginine.

11. Détergent contenant une protéase fortement alcaline selon l'une des revendications 1 à 5.

12. Détergent selon la revendication 11, contenant des constituants usuels de formulation pour détergents, tels que des tensioactifs, des adjuvants et des agents de blanchiment, et éventuellement d'autres additifs et adjuvants usuels en tant que constituants de détergents.
